# EUROPÄISCHE PATENTSCHRIFT

(11) **EP 0 934 329 B1**
(45) Veröffentlichungstag und Bekanntmachung des Hinweises auf die Patenterteilung: **12.12.2001**
(21) Anmeldenummer: 97942940.4
(22) Anmeldetag: 17.09.1997
(51) Int. Cl.: C07H 17/00, A61K 31/70

(54) **GLYCOKONJUGATE VON MODIFIZIERTEN CAMPTOTHECIN-DERIVATEN (20-O-VERKNÜPFUNG)**
GLYCOCONJUGATES FROM MODIFIED CAMPTOTHECIN DERIVATES (20-O-LINKAGE)
GLYCOCONJUGUES A BASE DE DERIVES MODIFIES DE CAMPTOTHECINE (20-O-LIAISON)

(30) Priorität: 30.09.1996 DE 19640206; 23.10.1996 DE 19643764
(43) Veröffentlichungstag der Anmeldung: 11.08.1999
(73) Patentinhaber: BAYER AG, 51368 Leverkusen (DE)
(72) Erfinder: LERCHEN, Hans-Georg, D-51375 Leverkusen (DE); VON DEM BRUCH, Karsten, D-51375 Leverkusen (DE); BAUMGARTEN, Jörg, D-42115 Wuppertal (DE); SPERZEL, Michael, D-42275 Wuppertal (DE)
(86) Internationale Anmeldenummer: EP9705088
(87) Internationale Veröffentlichungsnummer: WO9814459

(56) Entgegenhaltungen:
- EP-A- 0 640 622
- DE-A- 4 236 237
- GUPTA, ELORA ET AL: "Pharmacokinetic modulation of irinotecan and metabolites by cyclosporin A" CANCER RES. (1996), 56(6), 1309-14 CODEN: CNREA8;ISSN: 0008-5472, 15.März 1996, XP002053618
- CHEMICAL ABSTRACTS, vol. 126, no. 4, 27.Januar 1997 Columbus, Ohio, US; abstract no. 46340, KITAJIMA, MARIKO ET AL: "Production of camptothecinoids by utilizing cell cultures of Ophiorrhiza pumila and synthetic studies of their related compounds" XP002053619 & TENNEN YUKI KAGOBUTSU TORONKAI KOEN YOSHISHU (1996), 38TH, 283-288 CODEN: TYKYDS, 27.November 1996,

## Beschreibung

Die vorliegende Erfindung betrifft Glycokonjugate von Camptothecin-Derivaten, in denen mindestens eine Kohlenhydrat-Komponente über geeignete Spacer und Abstandshalter mit der 20-Hydroxylgruppe eines Camptothecin-Derivats verknüpft ist. Die Erfindung betrifft weiterhin Verfahren zur Herstellung der erfindungsgemäßen Verbindungen sowie deren Verwendung als Arzneimittel, insbesondere im Zusammenhang mit Krebserkrankungen.

20(S)-Camptothecin ist ein pentacyclisches Alkaloid, das 1966 von Wall et al. isoliert wurde (J.Am.Chem.Soc. 88, 3888 (1966)). Es besitzt ein hohes Antitumor-Wirkpotential in zahlreichen In-vitro- und in-vivo-Tests. Leider scheiterte jedoch die Realisierung des vielversprechenden Potentials in der Klinik an Toxizitäts- und Löslichkeitsproblemen.

Durch Öffnung des E-Ring-Lactons und Bildung des Natriumsalzes wurde eine wasserlösliche Verbindung erhalten, die in einem pH-abhangigen Gleichgewicht mit der ringgeschlossenen Form steht. Klinische Studien führten auch hier bisher nicht zum Erfolg.

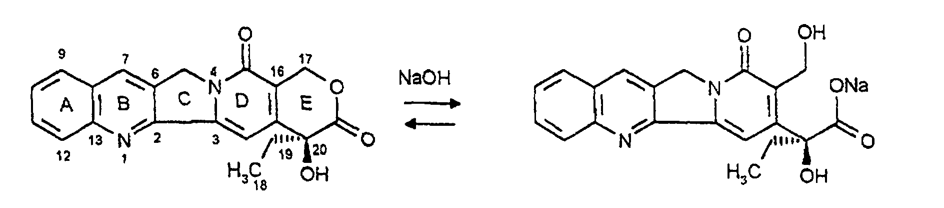

Etwa 20 Jahre später wurde gefunden, daß die biologische Aktivität auf eine Enzyminhibition der Topoisomerase I zuruckzuführen ist. Seither wurden die Forschungsaktivitäten wieder verstärkt, um verträglichere und in-vivo wirksame Camptothecin-Derivate zu finden.

Zur Verbesserung der Wasserlöslichkeit wurden Salze von A-Ring- und B-Ring-modifizierten Camptothecin-Derivaten sowie von 20-O-Acyl-Derivaten mit ionisierbaren Gruppen beschrieben (Vishnuvajjala et al. US 4943579). Letzteres Prodrug-Konzept wurde später auch auf modifizierte Camptothecin-Derivate übertragen (Wani et al. WO 9602546) Die beschriebenen 20-O-Acyl-Prodrugs haben allerdings in-vivo eine sehr kurze Halbwertszeit und werden sehr schnell zum Grundkörper gespalten.

Dokument EP-A-0 640 622 offenbart im allgemeinem Polysaccharidderivate von Wirkstoffen und die erhöhte Tumorselektivität dieser Verbindungen. Camptothecin ist in EP-A-0 640 622 als möglicher Wirkstoff an keiner Stelle erwähnt.

In Dokument DE-A-42 36 237 sind Substrat-Spacer-Wirkstoff Verbindungen und ihre Verwendung als Arzneimittel beschrieben, wobei der Substrat ein enzymatisch abspaltbares Kohlenhydrat ist. Jedoch enthält DE-A-42 36 237 kein Beispiel für eine entsprechende Campthotecin-Verbindung.

Wir fanden nun überraschend, daß die Anknüpfung von Kohlenhydratderivaten beispielsweise über Peptid-Spacer an die 20-Hydroxyl-Gruppe von A- und/oder B-Ring-modifizierten Camptothecin-Derivaten zu einer Verbindungsklasse mit hoch-interessanten Eigenschaften führt:
- Durch die esterartige Anknüpfung der Carrier-Reste an die 20-Hydroxylgruppe wird der für die Wirkung wichtige Lactonring der Camptothecin-Derivate stabilisiert.
- Die so erhaltenen Konjugate zeigen in-vitro eine hohe Aktivität gegenüber Tumorzellinien und Tumorxenografts.
- Sie weisen gegenüber den zugrundeliegenden Toxophoren eine deutlich höhere Verträglichkeit und Tumorselektivität sowie eine verbesserte Löslichkeit, insbesondere in wäßrigen Medien auf.
- In-vivo zeigen sie exzellente therapeutische Wirksamkeit über mehrere Dosisstufen
- Sie sind in extrazellularem Medium und in Blut wesentlich stabiler als die zuvor beschriebenen 20-O-Acyl-Prodrugs von Camptothecin.

Die Erfindung betrifft Verbindungen der allgemeinen Formel (I)

A-Cp-B (I)

worin
- Cp: für eine Gruppe der Formeln 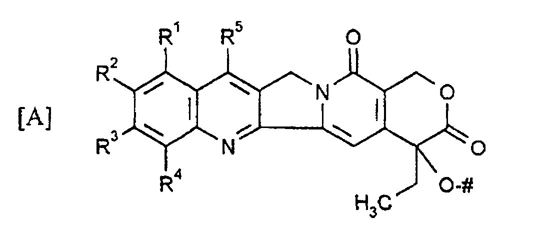 worin
R¹, R², R³ und R⁴ unabhängig voneinander Wasserstoff, Alkyl mit bis zu 3 Kohlenstoffatomen, Halogen, Amino, Hydroxy oder Nitro bedeuten können oder
R² und R³ zusammen eine Gruppe der Formel 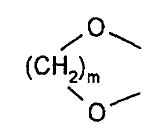 bedeuten, wobei
m die Werte 1 oder 2 annehmen kann, und
R⁵ für H, -CH₂CH₃, -CH₂-O-*, -CH₂-NH*, 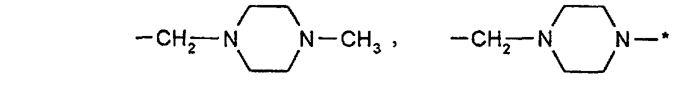 steht,
oder für -CH₂-N(CH₂CH₃)R⁶ oder 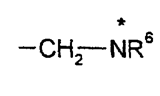 steht,
worin R⁶ Arylmethyl oder Hetarylmethyl bedeutet, 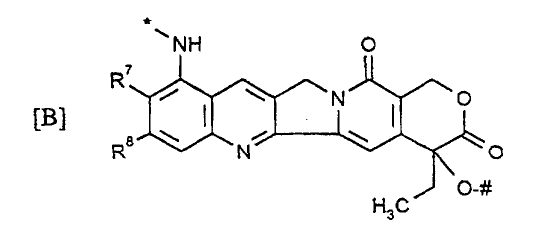 worin
R⁷ und R⁸ wie R² und R³ definiert sind und mit diesen gleich oder verschieden sein können, 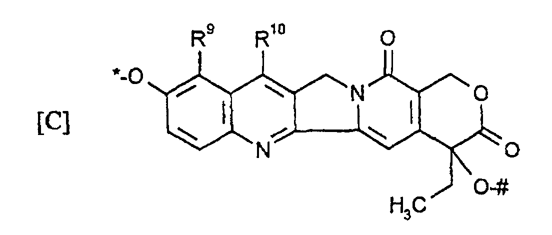 worin
R⁹ für Wasserstoff oder -CH₂-N(CH₃)₂ steht und
R¹⁰ für Wasserstoff oder Ethyl steht,
oder 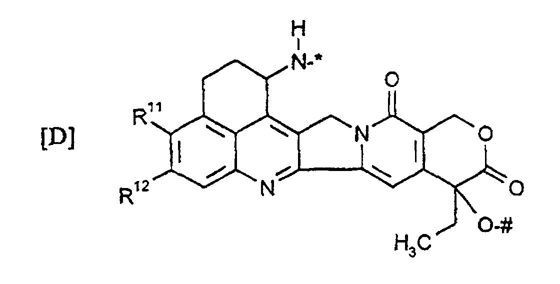 worin
R¹¹ und R¹² wie R² und R³ definiert sind und mit diesen gleich oder verschieden sein können,
oder 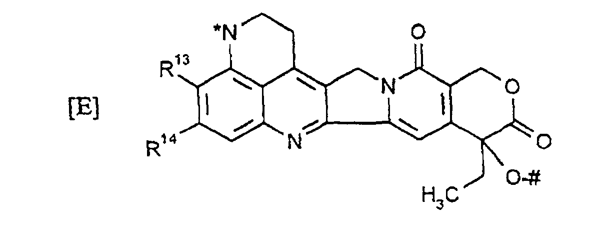 worin
R¹³ und R¹⁴ wie R² und R³ definiert sind und mit diesen gleich oder verschieden sein können, steht, wobei Cp an den mit # markierten Positionen mit A und an den mit * markierten Positionen mit B verknüpft ist,
- A: einen Rest der Formel 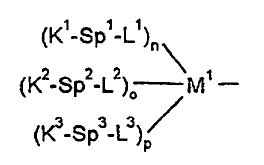 bedeutet, wobei gilt 1 ≤ (n + o + p) ≤ 3,
- B: Wasserstoff oder einen Rest der Formel 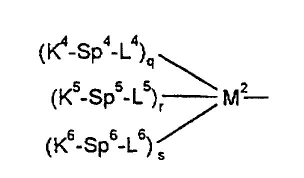 bedeutet, wobei gilt 0 ≤ (q + r + s) ≤ 3, worin

- M¹ und M²: unabhängig voneinander für eine Brücken-Gruppierung stehen, deren Hauptkette bis zu 21 Atome in linearer Abfolge umfaßt,
- L¹, L², L³, L⁴, L⁵ und L⁶: unabhängig voneinander für Linker-Gruppierungen stehen, wie sie in der Glycokonjugatchemie gängigerweise eingesetzt werden (siehe Übersichtsartikel Lee Y.C. und Lee R. in Lectins and Cancer 1991, 53-69, ed. by Gabius HJ and Gabius S., Springer-Verlag),
- Sp¹, Sp², Sp³, Sp⁴, Sp⁵ und Sp⁶: unabhängig voneinander für Arylen mit bis zu 10 Kohlenstoffatomen oder für Alkylen mit bis zu 8 Kohlenstoffatomen stehen, die jeweils gegebenenfalls substituiert sind, und
- K¹, K², K³, K⁴, K⁵ und K⁶: unabhangig voneinander für einen Rest der Formel (II) 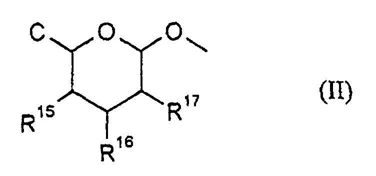
stehen, worin
- C: Methyl, Hydroxymethyl, Alkoxymethyl mit bis zu 6 Kohlenstoffatomen, Acyloxymethyl mit bis zu 6 Kohlenstoffatomen oder einen Rest der Formel -CH₂-D bedeutet, worin
- D: für einen Rest der Formel (II) steht,
- R¹⁵, R¹⁶ und R¹⁷: unabhängig voneinander Wasserstoff, Hydroxy, gegebenenfalls durch Hydroxyl substituiertes Alkoxy mit bis zu 6 Kohlenstoffatomen, gegebenenfalls durch Alkyl oder Acyl mit bis zu 6 Kohlenstoffatomen substituiertes Amino, Halogen, Sulfat oder eine Gruppe der Formeln 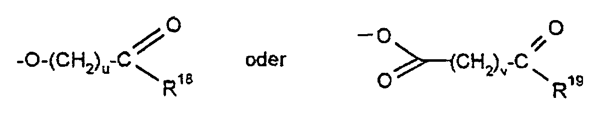 bedeuten, worin

- R¹⁸ und R¹⁹: unabhängig voneinander für Hydroxyl oder Alkoxy mit bis zu 6 Kohlenstoffatomen stehen oder für Amino, das gegebenenfalls durch Alkyl mit bis zu 6 Kohlenstoffatomen substituiert ist, stehen und
- u und v: unabhängig voneinander die Werte 0, 1, 2, 3 oder 4, insbesondere die Werte 1, 2, 3 oder 4, annehmen können
oder
- R¹⁵, R¹⁶ und R¹⁷: unabhängig voneinander für einen Rest der Formel (II) stehen
oder
zwei der Reste R¹⁵, R¹⁶, R¹⁷ zusammen für eine Epoxygruppe stehen,
sowie deren Isomere, Isomerengemische und Salze
mit der Maßgabe, dass R¹, R², R³, R⁴ und R⁵ nicht gleichzeitig Wasserstoff sein können.

Der Begriff "Alkylgruppen" soll, sofern nicht anders angegeben im Sinne der Erfindung geradkettige, verzweigte, cyclische und Cycloalkylreste enthaltende Alkylreste umfassen. Diese Definition soll sinngemäß auch für alle anderen Alkylgruppen enthaltenden Reste gelten, wie beispielsweise Alkoxy, Acyl usw..

Die bei der Definition von R⁶ angegebenen Begriffe Arylmethyl und Hetarylmethyl können beispielsweise Phenylmethyl bzw. Pyridylmethyl bedeuten.

Bevorzugt sind Verbindungen der allgemeinen Formel (I), worin K¹, K², K³, K⁴, K⁵ und K⁶ unabhängig voneinander für einen Rest der Formel (II) stehen können, wobei
- c: Methyl, Hydroxymethyl, Methoxymethyl oder Acetoxymethyl bedeutet,
- R¹⁵: Wasserstoff, Hydroxyl, Methoxy oder eine Gruppe der Formeln 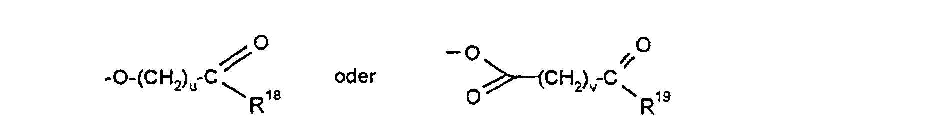 bedeutet, worin

- u und v: unabhängig voneinander die Werte 1 oder 2 annehmen können und
- R¹⁸ und R¹⁹: unabhängig voneinander für Hydroxyl oder Alkoxy mit bis zu 4 Kohlenstoffatomen stehen,
oder
- R¹⁵: für einen Rest der Formel (II) steht,
- R¹⁶: Wasserstoff, Hydroxyl, Halogen, Alkoxy mit bis zu 4 Kohlenstoffatomen, Sulfat oder eine Gruppe der Formeln 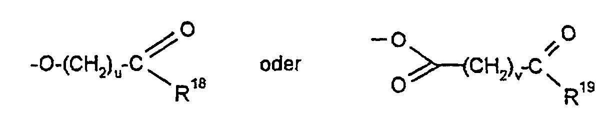 bedeutet, worin
u und v unabhängig voneinander die Werte 1 oder 2 annehmen können und
R¹⁸ und R¹⁹ unabhängig voneinander für Hydroxy oder Alkoxy mit bis zu 4 Kohlenstoffatomen stehen oder für Amino, das gegebenenfalls durch Alkyl mit bis zu 4 Kohlenstoffatomen substituiert ist, stehen,
- R¹⁷: Hydroxyl, Alkoxy mit bis zu 4 Kohlenstoffatomen, das gegebenenfalls durch Hydroxy substituiert ist, gegebenenfalls durch Alkyl oder Acyl mit bis zu 4 Kohlenstoffatomen substituiertes Amino, oder eine Gruppe der Formeln 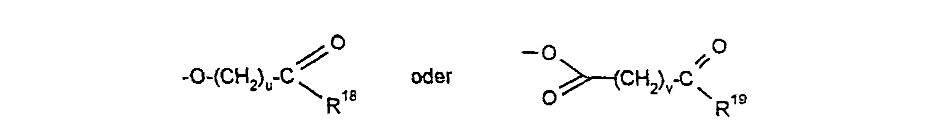
bedeutet, worin
- u und v: unabhängig voneinander die Werte 1 oder 2 annehmen können und
- R¹⁸ und R¹⁹: unabhängig voneinander für Hydroxyl oder Alkoxy mit bis zu 4 Kohlenstoffatomen stehen,
oder worin
- R¹⁵ und R¹⁶: gemeinsam für eine Epoxygruppe stehen,
sowie deren Isomere, Isomerengemische und Salze

Ganz besonders bevorzugt stehen K¹, K², K³, K⁴, K⁵ und/oder K⁶ für einen Rest der Formel (II), wobei
- C: Methyl, Hydroxymethyl, Methoxymethyl oder Acetoxymethyl bedeutet
- R¹⁵ und R¹⁷: jeweils ein Hydroxylgruppe bedeuten und
- R¹⁶: Wasserstoff, Hydroxyl, Halogen, Alkoxy mit bis zu 4 Kohlenstoffatomen, Sulfat oder eine Gruppe der Formeln 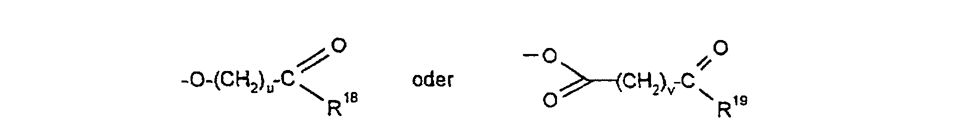 bedeutet, worin
u und v unabhangig voneinander die Werte 1 oder 2 annehmen können und
- R¹⁸ und R¹⁹: unabhängig voneinander für Hydroxy oder Alkoxy mit bis zu 4 Kohlenstoffatomen stehen oder für Amino, das gegebenenfalls durch Alkyl mit bis zu 4 Kohlenstoffatomen substituiert ist, stehen.

Gemäß einer besonders bevorzugten Ausführungsform umfassen die KohlenhydratBausteine K¹, K², K³, K⁴, K⁵ und/oder K⁶ jeweils maximal zwei Monosaccharid-Bausteine.

Weiterhin bevorzugt sind Verbindungen der allgemeinen Formel (I), worin Sp¹, Sp², Sp³, Sp⁴, Sp⁵ und/oder Sp⁶ unabhängig voneinander für Arylen mit bis zu 10 Kohlenstoffatomen stehen können, das mit je einer Gruppe K¹, K², K³, K⁴, K⁵ oder K⁶ und L¹, L², L³, L⁴, L⁵ oder L⁶ verknüpft ist und das gegebenenfalls noch ein- oder mehrfach durch Hydroxy, Carboxy, Carboxyalkyl mit bis zu 4 Kohlenstoffatomen, Nitro, Cyano, Halogen, Alkyl mit bis zu 4 Kohlenstoffatomen, Halogenalkyl mit bis zu 4 Kohlenstoffatomen oder durch Alkoxy mit bis zu 4 Kohlenstoffatomen substituiert ist, sowie deren Isomere, Isomerengemische und Salze.

Besonders bevorzugt sind Sp¹, Sp², Sp³, Sp⁴, Sp⁵ und/oder Sp⁶ abgesehen von K¹, K², K³, K⁴, K⁵ oder K⁶ und L¹, L², L^{3,} L⁴, L⁵ oder L⁶ unsubstituiert oder gegebenenfalls substituiert durch Halogen, Nitro, Alkyl mit bis zu 6 Kohlenstoffatomen, Alkoxy mit bis zu 2 Kohlenstoffatomen, -OCF₃ und/oder CF₃.

Ganz besonders bevorzugt sind Sp¹, Sp², Sp³, Sp⁴, Sp⁵ und/oder Sp⁶ paraständig mit je einer Gruppe K¹, K², K³, K⁴, K⁵ oder K⁶ und L¹, L², L³, L⁴, L⁵ oder L⁶ verknüpft und tragen keine weiteren Substituenten.

Weiterhin bevorzugt sind Verbindungen der allgemeinen Formel (I)
worin
L¹, L², L³, L⁴, L⁵ und L⁶ unabhängig voneinander 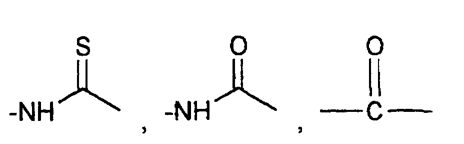 oder 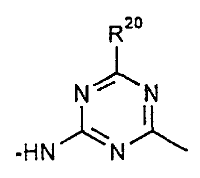 bedeuten, wobei
   R²⁰ für Chlor oder für Hydroxyalkylamino mit bis zu 6 Kohlenstoffatomen steht.

Besonders bevorzugt stehen L¹, L², L³, L⁴, L⁵ und L⁶ für

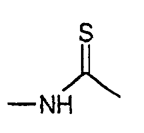

Weiterhin bevorzugt sind Verbindungen der allgemeinen Formel (I), worin M¹ und M² unabhängig voneinander für ein Peptid stehen können, das über eine Aminofunktion mit L¹, L², L³, L⁴, L⁵ und/oder L⁶ verknüpft ist, über eine Acylfunktion mit Cp verknüpft ist und dessen Aminosäurebausteine gegebenenfalls Schutzgruppen tragen können. Besonders bevorzugt sind Mono-, Di- und Tripeptide, insbesondere Mono- und Dipeptide.

Die Aminosäurebausteine werden bevorzugt ausgewählt aus der Gruppe Glycyl, Alanyl, Valyl, Leucyl, Lysyl, Seryl, Glutamyl, Threonyl, Asparagyl, Isoleucyl, Diaminopropionyl, Diaminobutyryl, Arginyl, Histidyl und/oder Ornithyl.

Besonders bevorzugt sind die Aminosäurebausteine Glycyl, Alanyl, Valyl, Leucyl, Lysyl, Seryl, Asparagyl, Histidyl und/oder Glutamyl.

Die erfindungsgemäßen Verbindungen können in stereoisomeren Formen, beispielsweise als Enantiomere oder Diastereomere, oder als deren Gemische, beispielsweise als Racemat, vorliegen. Die Erfindung betrifft sowohl die reinen Stereoisomere als auch deren Gemische

Stereoisomerengemische können, falls erforderlich, in bekannter Weise in die stereoisomer einheitlichen Bestandteile getrennt werden, beispielsweise durch Chromatographie oder durch Kristallisationsverfahren

Die Stereochemie am anomeren Zentrum der Kohlenhydratbausteine K¹, K², K³, K⁴, K⁵ und/oder K⁶ kann α oder β sein Weiterhin konnen sie in der D- oder L-Form vorliegen. Durch die Stereochemie an den anderen Zentren kann sich die gluco-, manno-, galacto-, gulo-, rhamno- oder fuco-Konfiguration ergeben.

Die Aminosaurebausteine können jeweils in der D- oder in der L-Form vorliegen.

Der Camptothecin-Baustein Cp kann in der 20(R)- oder in der 20(S)-Konfiguration oder als Gemisch dieser beiden steroisomeren Formen vorliegen. Bevorzugt ist die 20(S)-Konfiguration.

Weiterhin können bedingt durch eine Rotationshinderung die erfindungsgemäßen Verbindungen in rotationsisomeren Formen oder als deren Gemisch auftreten. Die Erfindung betrifft sowohl die reinen Rotationsisomere als auch deren Gemische.

Rotationsisomerengemische können gegebenenfalls, falls erforderlich mittels bekannten Methoden in die einheitlichen Bestandteile getrennt werden beispielsweise durch Chromatographie (z.B. HPLC) oder durch Kristallationsverfahren. Möglich ist dies auf der Endstufe und gegebenenfalls auch auf Zwischenstufen Aus den rotamerenreinen Zwischenstufen können gegebenenfalls durch geeignete Syntheseführung die rotamerenreinen Endstufen hergestellt werden.

Bevorzugte Beispiele für den Camptothecin-Baustein sind:

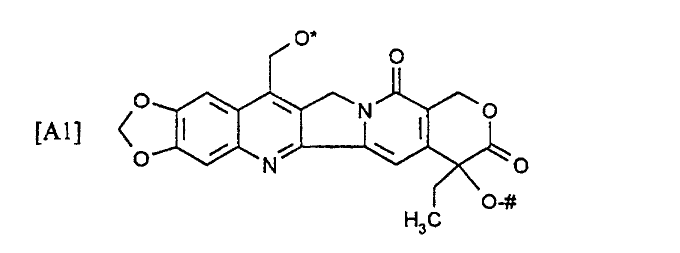

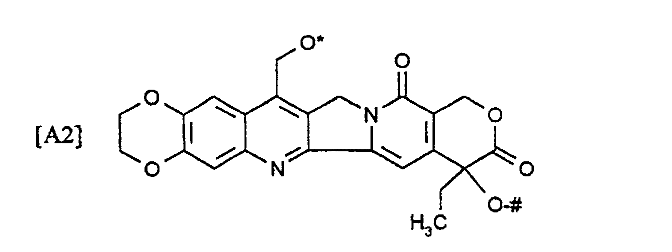

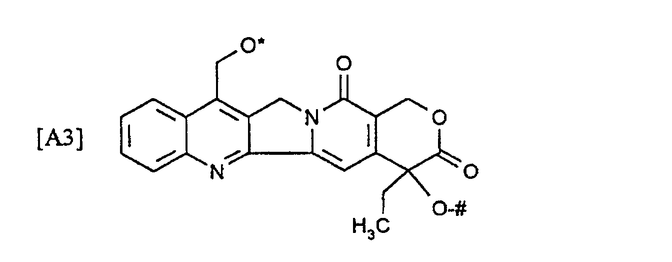

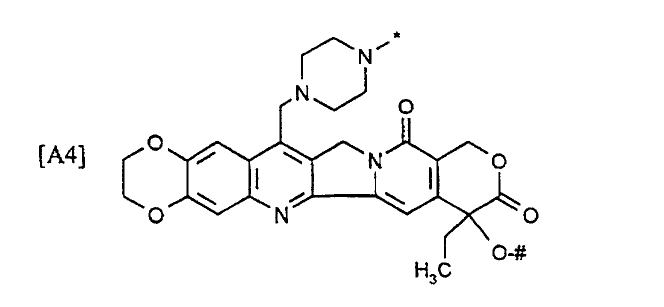

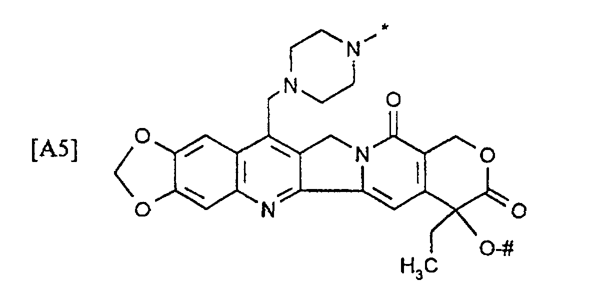

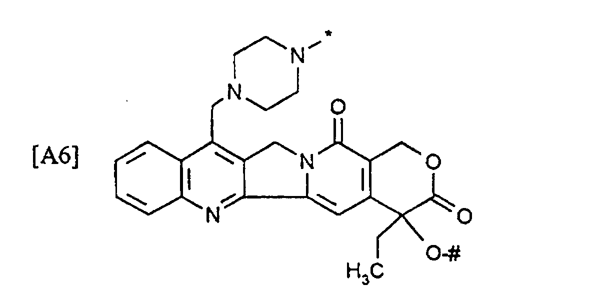

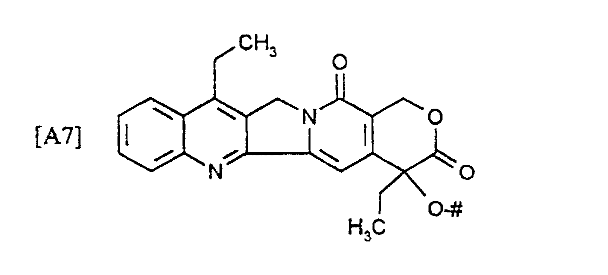

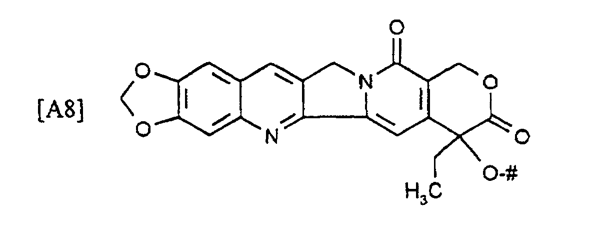

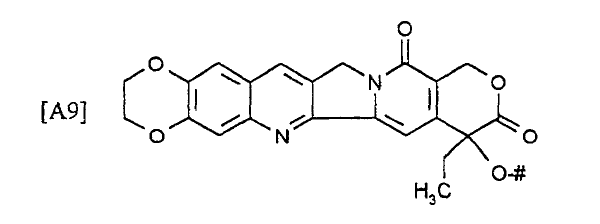

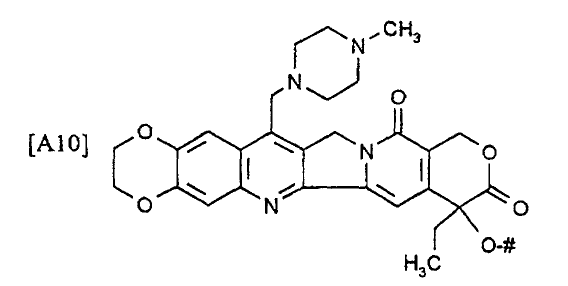

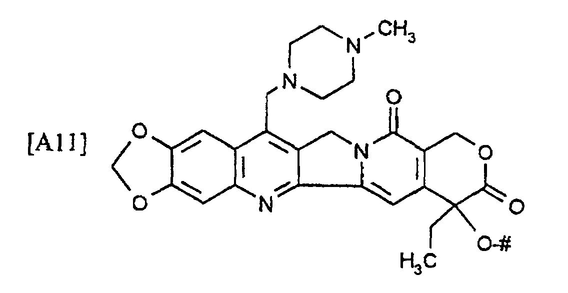

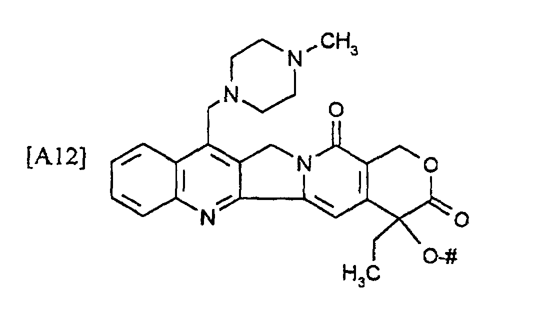

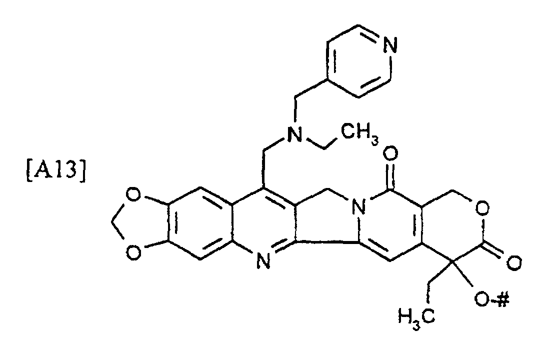

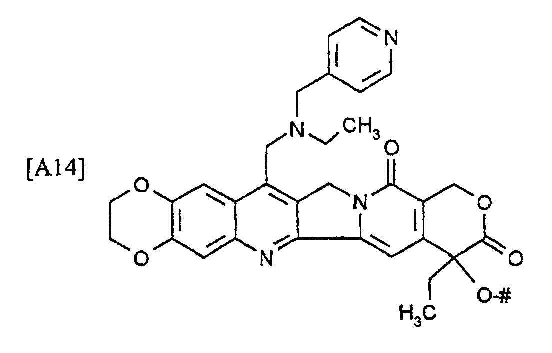

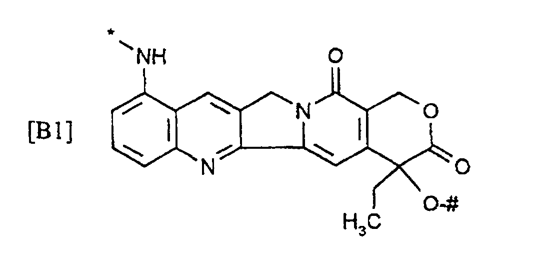

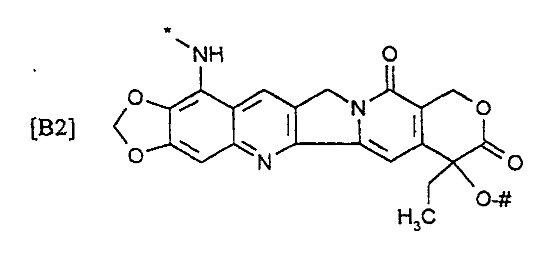

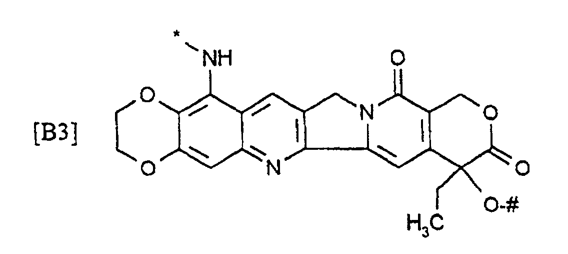

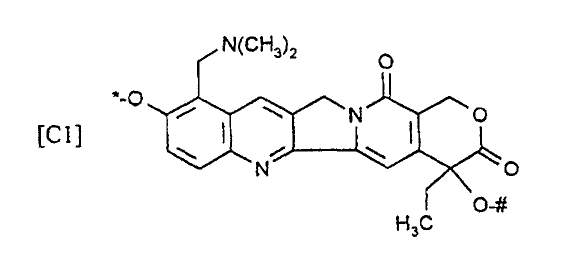

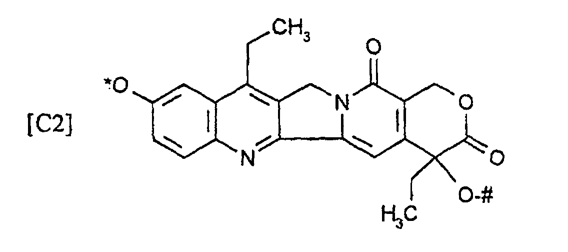

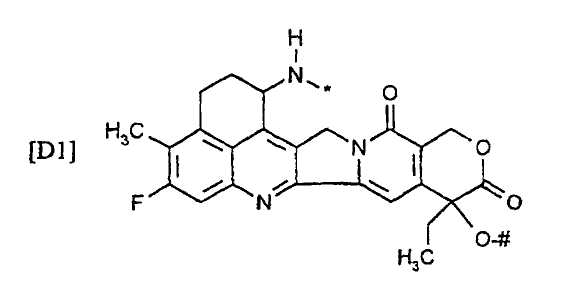

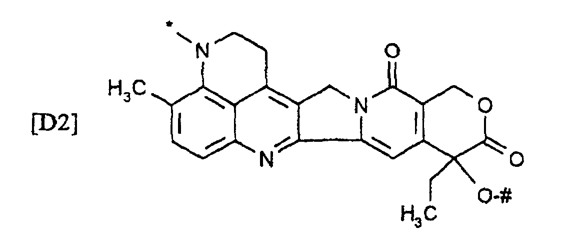

Von diesen Beispielen besonders bevorzugt sind [A3], [A7], [A8], [A9], [A10], [A11], [A14], [B1], [B2], [C2] und [D1].

Durch Kombination der für die einzelnen Reste angegebenen bevorzugten oder besonders bevorzugten Bedeutungen ergeben sich entsprechend ganz besonders bevorzugte Verbindungen der allgemeinen Formel (I)

Die erfindungsgemäßen Verbindungen können auch in Form ihrer Salze vorliegen. Im allgemeinen seien hier Salze mit organischen oder anorganischen Basen oder Säuren sowie innere Salze genannt.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsauren, wie z.B. Essigsäure, Trifluoressigsäure, Maleinsaure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsaure, 1,5-Naphthalindisulfonsäure oder Camphersulfonsaure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di-bzw Triethanolamin, Dicyclohexylamin, Dimethylaminoethanol, Arginin, Lysin, Ethylendiamin oder Phenethylamin.

Die erfindungsgemäßen Glycokonjugate können beispielsweise hergestellt werden durch Verknüpfung von modifizierten Camptothecin-Derivaten mit aktivierten Carboxylkomponenten, die ihrerseits Teile von geschützten Aminosäuren, Peptiden oder kohlenhydratmodifizierten Peptiden darstellen können.

Die Erfindung betrifft daher weiterhin ein Verfahren zur Herstellung von Verbindungen der allgemeinen Formel (I), dadurch gekennzeichnet, daß man Verbindungen der allgemeinen Formel (III)

H_{B}-Cp-H_{A} (III)

worin Cp die oben angegebene Bedeutung hat und das Wasserstoffatom H_{A} an der mit # und H_{B} an der mit * bezeichneten Position sitzt, gegebenenfalls nach Ersatz von H_{B} durch eine Schutzgruppe, mit einer dem oben definierten Rest M¹ entsprechenden aktivierten Carboxylkomponente M¹a, die gegebenenfalls Schutzgruppen trägt, in einem geeigneten Losungsmittel, gegebenenfalls in Gegenwart einer Base, nach üblichen Methoden umsetzt, gegebenenfalls mittels bekannter Methoden selektiv eine, mehrere oder alle Schutzgruppen von M¹ abspaltet und das Produkt mit Verbindungen der allgemeinen Formel (IV)

K¹-Sp¹-L¹a (IV)

worin K¹ und Sp¹ wie oben definiert sind und L¹a für eine reaktive Vorstufe der Gruppe L¹ steht, umsetzt, wobei gegebenenfalls die Schutzgruppen selektiv abgespalten und schrittweise verschiedene Gruppen K²-Sp²-L²- und K³-Sp³-L³- in vergleichbarer Weise eingeführt werden können, und daß man, falls mit der mit * bezeichneten Position eine Kohlenhydrat-Komponente verknüpft werden soll, gegebenenfalls selektiv die Schutzgruppe, die H_{B} ersetzt, mittels bekannter Methoden abspaltet, und in der oben beschriebenen Weise den Rest M² und wie gewünscht Reste der Formeln K⁴-Sp⁴-L⁴-, K⁵-Sp⁵-L⁵- und K6-Sp6-L6- einführt
oder daß man, falls M¹ und/oder M² ein Peptid bedeuten, in vergleichbarer Weise nach üblichen Methoden einen ersten Aminosäurerest in Form der entsprechenden gegebenenfalls Schutzgruppen tragenden Carboxylkomponente einführt, gegebenenfalls Schutzgruppen abspaltet, weiterhin gegebenenfalls Schutzgruppen tragende Aminosäurereste anknüpft, gegebenenfalls erneut Schutzgruppen abspaltet wie oben angegeben Reste der Formeln K¹-Sp¹-L¹-, K²-Sp²-L²-, K³-Sp³-L³-, K⁴-Sp⁴-L⁴-, K⁵-Sp⁵-L⁵- und/oder K⁶-Sp⁶-L⁶- einführt und falls erforderlich Schutzgruppen abspaltet.

Die Reaktionen können bei verschiedenen Druck- und Temperaturverhältnissen, beispielsweise 0,5 bis 2 bar, bzw. -30 bis +100°C, in geeigneten Lösungsmitteln wie Dimethylformamid (DMF), Tetrahydrofuran (THF), Dichlormethan, Chloroform, niederen Alkoholen, Acetonitril, Dioxan, Wasser oder in Gemischen der genannten Lösungsmittel durchgeführt werden. In der Regel sind Reaktionen in DMF oder THF/Dichlormethan bei Raumtemperatur und Normaldruck bevorzugt.

Für die Aktivierung der Carboxylgruppen kommen die in der Peptidchemie bekannten Kupplungsreagenzien wie sie z.B. in Jakubke/Jeschkeit: Aminosäuren, Peptide, Proteine; Verlag Chemie 1982 oder Tetrahedr. Lett. 34, 6705 (1993) beschrieben sind, in Frage. Bevorzugt sind beispielsweise Säurechloride, N-Carbonsäureanhydride oder gemischte Anhydride.

Weiterhin geeignet zur Aktivierung der Carboxylgruppen ist die Bildung von Addukten mit Carbodiimiden z.B. N,N'-Diethyl-, N,N'-Diisopropyl-, N,N'-Dicyclohexylcarbodiimid, N-(3-Dimethylaminopropyl)-N'-ethyl-carbodiimid-Hydrochlorid, N-Cyclohexyl-N'-(2-morpholinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsaureanhydrid, oder Isobutylchloroform, oder Benzotriazolyloxy-tris(dimethylamino)phosphonium-hexafluorophosphat, 1-Hydroxybenzotriazol- oder Hydroxysuccinimidester. Weiterhin kann die Aminosäurekomponente auch in Form eines Leuchs'schen Anhydrids eingesetzt werden.

Als Basen können beispielsweise Triethylamin, Ethyl-diisopropylamin, Pyridin, N,N-Dimethylaminopyridin oder andere eingesetzt werden.

Als Schutzgruppen für eventuelle weitere reaktive Funktionen im Camptothecin-Teil oder für Drittfunktionen der Aminosäuren können die in der Peptidchemie bekannten Schutzgruppen beispielsweise vom Urethan-, Alkyl-, Acyl-, Ester- oder Amid-Typ eingesetzt werden.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, tert-Butoxycarbonyl (Boc), Allyloxycarbonyl, Vinyloxycarbonyl, 3,4,5-Trimethoxybenzyloxycarbonyl, Phthaloyl, 2,2,2-Trichlorethoxycarbonyl, 2,2,2-Trichlor-tert-butoxycarbonyl, Menthyloxycarbonyl, 4-Nitrophenoxycarbonyl, Fluorenyl-9-methoxycarbonyl (Fmoc), Formyl, Acetyl, Propionyl, Pivaloyl, 2-Chloracetyl, 2-Bromacetyl, 2,2,2-Trifluoracetyl, 2,2,2-Trichloracetyl, Benzoyl, Benzyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl oder 2-Nitrophenylsulfenyl. Besonders bevorzugt sind die Fmoc-Gruppe und die Boc-Gruppe.

Bevorzugte Carboxylschutzgruppen sind lineare oder verzweigte C₁-C₄-Alkylester.

Die Modifizierung der mit einer Brücken-Gruppierung M¹ und/oder M² verknüpften Camptothecin-Derivate mit Kohlenhydratresten kann über verschiedene Methoden und Linkergruppen erfolgen Bevorzugt ist z.B. die Überführung von p-Amino-phenylglycosiden in Isothiocyanate und die Verknüpfung beispielsweise mit Aminogruppen. Weiterhin können auch Carboxyalkyl- oder Amino-alkylglycoside leicht mit Amino- bzw. Carboxylgruppen gekuppelt werden.

Die Abspaltung von Schutzgruppen in entsprechenden Reaktionsschritten kann zum Beispiel durch Säure- oder Base-Einwirkung, hydrogenolytisch oder auf andere Weise reduktiv erfolgen.

### Biologische Testung

### 1. Wachstumsinhibitionstest zur Bestimmung der cytotoxischen Eigenschaften:

Die humanen Dickdarmzellinien SW 480 und HT 29 (ATCC-Nr. CCL 228 und HBT-38) sowie die Maus-Melanom-Zellinie B16F10 wurden in Rouxschalen in RPMI 1640 Medium unter Zusatz von 10 % FCS gezogen. Anschließend wurde trypsiniert und in RPMI plus 10 % FCS zu einer Zellzahl von 50.000 Zellen/ml aufgenommen. 100 µl Zellsuspension/Well wurden in eine 96 Mikrowellplatte gegeben und 1 Tag bei 37°C im CO₂-Brutschrank inkubiert. Anschließend wurden weitere 100 µl RPMI Medium und 1 µl DMSO mit den Prüfsubstanzen zugesetzt. Das Wachstum wurde nach Tag 3 und Tag 6 überprüft. Dazu wurde zu jedem Mikrowell 40 µl MTT-Losung (3-(4,5-Dimethylthiazol-2-yl)-2,5-diphenyltetrazolinbromid) mit einer Ausgangskonzentration von 5 mg/ml H₂O zugesetzt. Es wurde 5 Stunden im CO₂-Brutschrank bei 37°C inkubiert. Anschließend wurde das Medium abgesaugt und 100 µl i-Propanol/Well zugesetzt. Nach 30 min Schütteln mit 100 µl H₂O wurde die Extinktion bei 540 nm mit einem Titertek Multiskan MCC/340 (Flow) gemessen.

Die cytotoxische Wirkung ist in der Tabelle 1 als IC₅₀-Wert jeweils für die SW 480- und HT 29- und B16F10-Zellinie angegeben:

**Tabelle 1:**

| Beispiel | IC₅₀ / nM SW 480 | IC₅₀ / nM HT 29 | IC₅₀ / nM B16F10 |
|---|---|---|---|
| 1.1 | 15 | 10 | 30 |
| 1.5 | 100 | 60 | 300 |
| 2.1 | 7 | 5 | 15 |
| 2.3 | 6 | 3 | 9 |
| 2.4 | 5 | 3 | 10 |
| 3.1 | 10 | 8 | 150 |
| 4.1 | 40 | 40 | 300 |
| 5.1 | 70 | 40 | 200 |
| 6.1 | 150 | 200 | 3000 |

### 2. Hämatopoetische Aktivität von Glycokonjugaten im Vergleich zum zugrundeliegenden Wirkstoff:

### Material und Methoden:

Knochenmarkzellen wurden aus dem Femur der Maus gespült 10⁵-Zellen wurden in McCoy 5A-Medium (0,3 % Agar) zusammen mit rekombinanten murinen GM-CSF (Genzyme; Stammzellen-Kolonienbildung) und den Substanzen (10⁻⁴ bis 100 µg/ml) bei 37°C und 7% CO₂ inkubiert. 7 Tage später wurden die Kolonien (<50 Zellen) und Kluster (17-50 Zellen) ausgezählt.

### Ergebnisse:

Wie in Tab. 2 dargestellt, zeigen die untersuchten Glycokonjugate eine gegenüber dem zugrundeliegenden Wirkstoff drastisch verminderte Hemmung der Knochenmarkstammzellproliferation.

**Tabelle 2:**

| Hemmung der CSF-induzierten Proliferation von Knochenmarkstammzellen der Maus | |
|---|---|
| Beispiel | IC₅₀ [µg/ml] |
| 7-Ethyl-Camptothecin | 2 x 10⁻⁶ |
| 1.1 | 2 x 10⁻³ |
| 9-Amino-Camptothecin | 1 x 10⁻³ |
| 3.1 | 2 x 10⁻² |

### 3. In-vivo-Hemmung des Tumorwachstums im Nacktmaus-Modell

### Material:

Für alle in-vivo-Experimente zur Untersuchung der Hemmung des Tumorwachstums wurden athymische Nacktmäuse (NMRI nu/nu-Stamm) verwendet. Das ausgewählte großzellige Lungenkarzinom LXFL 529 wurde durch serielle Passage in Nacktmäusen entwickelt. Der menschliche Ursprung des Tumors wurde durch isoerizymatische und immunohistochemische Methoden belegt.

### Experimenteller Aufbau:

Der Tumor wurde subcutan in beide Flanken von 6 bis 8 Wochen alten nu/nu-Nacktmäusen implantiert. Die Behandlung wurde, abhängig von der Verdopplungszeit, gestartet sobald die Tumoren einen Durchmesser von 5-7 mm erreicht hatten. Die Mäuse wurden der Behandlungsgruppe und der Kontrollgruppe (5 Mäuse pro Gruppe mit 8 - 10 auswertbaren Tumoren) durch Randomisieren zugeteilt. Die einzelnen Tumoren der Kontrollgruppe wuchsen alle progressiv.

Die Größe der Tumoren wurde in zwei Dimensionen mittels Schieblehre vermessen. Das Tumorvolumen, das gut mit der Zellzahl korreliert, wurde anschließend für alle Auswertungen verwendet. Das Volumen wurde gemäß der Formel "Länge x Breite x Breite / 2" berechnet ([a x b²] / 2, a bzw. b stehen für zwei rechtwinklig angeordnete Durchmesser).

Die Werte des relativen Tumorvolumens (RTV) wurden für jeden einzelnen Tumor durch Dividieren der Tumorgröße am Tag X mit der Tumorgröße des Tages 0 (zum Zeitpunkt der Randomisierung) berechnet. Die mittleren Werte des RTV wurden dann für die weitere Auswertung verwendet.

Die Hemmung der Zunahme des Tumorvolumens (Tumorvolumen der Test-gruppe/Kontrollgruppe, T/C, in Prozent) war der abschließende Meßwert.

### Behandlung:

Die Applikation der Verbindungen erfolgte an Tag 1, 2 und 3 nach Randomisierung intraperitoneal (i.p).

### Ergebnisse:

Anhand der Verbindung aus Beispiel 1.1 ist die therapeutische Wirksamkeit der erfindungsgemäßen Glycokonjugate gegenüber dem großzelligen humanen Lungentumorxenografts LXFL 529 dargestellt. Die Therapie führt bei der maximal tolerablen Dosis (MTD) und bei 1/2 MTD zu deutlichen Tumorremission.

**Tabelle 3:**

| Therapie | Dosis [mg/kg/ Tag] | Überlebenszeit [Tage] | | Zahl der Tumoren | relatives Tumorvolumen an Tag 14 [% des Tages 0] | relatives Körpergewicht an Tag 7 [% des Tages 0] |
|---|---|---|---|---|---|---|
| Kontrollgruppe | - | >21 | >21 | | 692 | 102 |
| | | >21 | >21 | | | |
| | | >21 | | | | |
| 1.1 | 12,5 (MTD) | >21 | >21 | 9 | 0,1 | 95 |
| | | >21 | >21 | | | |
| | | >21 | | | | |
| 1.1 | 6,25 | >21 | >21 | 8 | 32 | 98 |
| | | >21 | >21 | | | |
| | | 13 | | | | |

Die erfindungsgemäßen Verbindungen weisen sowohl in-vitro als auch in-vivo eine überraschend starke Antitumor-Wirksamkeit gegenüber verschiedenen Tumoren, insbesondere solchen der Lunge und des Dickdarms, verbunden mit einer großen Selektivität gegenüber nicht malignen Zellen auf.

Sie eignen sich daher zur Behandlung von Krebserkrankungen insbesondere von solchen der Lungen und des Dickdarms.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgefuhrten pharmazeutischen Zubereitungen können außer den erfindungsgemäßen Verbindungen auch weitere pharmazeutische Wirkstoffe enthalten

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemaßen Wirkstoffe vorzugsweise in Mengen von etwa 1 bis etwa 80, insbesondere 3 bis 30mg/kg Körpergewicht

### Beispiele

### Kohlehydrat-Edukte

### Beispiel I.1:

### p-Aminophenyl-3-O-methyl-β-L-fucopyranosid

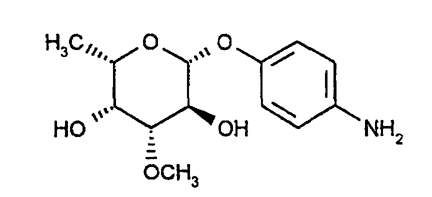

### I.1.a) p-Nitrophenyl-3-O-methyl-β-L-fucopyranosid:

6 g (21 mmol) p-Nitrophenyl-β-L-fucopyranosid in 300 ml absol. Methanol werden mit 7,84 g (31,5 mmol) Dibutylzinnoxid versetzt und 2 h unter Rückfluß erhitzt. Anschließend wird eingeengt, der Rückstand getrocknet und dann in 300 ml DMF aufgenommen. Nach Zugabe von 15,7 ml Methyliodid wird der Ansatz 40 h bei 70°C gerührt. Das Lösungsmittel wird im Vakuum entfernt und der Rückstand in 300 ml Dichlormethan aufgenommen. Die Suspension wird filtriert, die verbleibende Lösung erneut eingeengt und einer Flash-Chromatographie (Dichlormethan/Methanol 99:1) unterzogen. Nach Einengen erhält man 3,82 g (61%) des Zielproduktes.

### I.1) p-Aminophenyl-3-O-methyl-β-L-fucopyranosid:

3,81 g (12,73 mmol) p-Nitrophenyl-3-O-methyl-β-L-fucopyranosid werden in Methanol gelöst und nach Zusatz von Platindioxid in einer Wasserstoffatmosphäre bei geringem Überdruck hydriert. Nach Abfiltrieren des Katalysators und Fällen mit Ether erhält man 3 g (88 %) des Zielprodukts. [DC: Dichlormethan/Methanol 9:1 R_{f}= 0,53].

### Beispiel L2:

### p-Aminophenyl-3-O-carboxymethyl-β-L-fucopyranosid

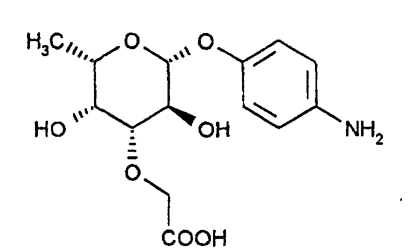

### 1.2.a) p-Nitrophenyl-3-O-methoxycarbonylmethyl-β-L-fucopyranosid:

1 g (3,5 mmol) p-Nitrophenyl-β-L-fucopyranosid und 1,3 g (5,2 mmol) Dibutylzinnoxid werden in 50 ml Methanol 2 h unter Rückfluß erhitzt. Die Lösung wird eingeengt, der Rückstand in 50 ml Dioxan aufgenommmen, mit 2 ml Bromessigsaure-methylester sowie 100 mg Tetrabutylammoniumiodid versetzt und 16 h unter Rückfluß erhitzt. Das Lösungsmittel wird abgedampft und das Produkt durch Flash-Chromatographie (Dichlormethan/Methanol 99:1) gereinigt. Nach Einengen der entsprechenden Fraktionen und Fällen aus Methanol/Ether erhält man 455 mg (37 %) der Zielverbindung.

### L2) p-Aminophenyl-3-O-carboxymethyl-β-L-fucopyranosid:

282 mg (0,79 mmol) p-Nitrophenyl-3-methoxycarbonylmethyl-β-L-fucopyranosid werden in 20 ml Methanol gelöst und mit 440 µl einer wäßrigen 2N Lithiumhydroxid-Lösung versetzt. Nach 2 h Rühren bei Raumtemp. wird mit saurem Ionenaustauscher SC108 auf pH 3 eingestellt und filtriert. Zum Filtrat werden 250 mg Palladium auf Aktivkohle zugesetzt. Anschließend wird 1,5 h mit Wasserstoff bei geringem Überdruck hydriert, der Katalysator abgetrennt und mit Methanol gewaschen. Einengen, Aufnehmen in Wasser und Gefriertrocknung führen in 86 %iger Ausbeute zum Zielprodukt (212 mg). [DC: Acetonitril/Wasser/Eisessig 5:1:0,2 R_{f}= 0,24]

Als weitere Kohlenhydratbausteine können beispielsweise die folgenden Derivate eingesetzt werden:
p-Aminophenyl-β-L-fucopyranosid
p-Aminophenyl-2-O-methyl-β-L-fucopyranosid
p-Aminophenyl-2-O-hydroxyethyl-β-L-fucopyranosid
p-Aminophenyl-4-O-methyl-β-L-fucopyranosid
p-Aminophenyl-3-O-methyl-α-L-fucopyranosid
p-Aminophenyl-3-O-n-propyl-β-L-fucopyranosid
p-Aminophenyl-3-desoxy-β-L-fucopyranosid
p-Aminophenyl-3,4-didesoxy-β-L-fucopyranosid
p-Aminophenyl-3,4-epoxy-β-L-fucopyranosid
p-Aminophenyl-4-desoxy-β-L-fucopyranosid
p-Aminophenyl-3-O-methoxycarbonylmethyl-β-L-fucopyranosid
p-Aminophenyl-3-O-hydroxyethyl-β-L-fucopyranosid
p-Aminophenyl-2-O-carboxymethyl-β-L-fucopyranosid
p-Aminophenyl-3-O-succinyl-β-L-fucopyranosid
p-Aminophenyl-3,4-di-O-methyl-β-L-fucopyranosid
p-Aminophenyl-3-O-carbamoylmethyl-β-L-fucopyranosid
p-Aminophenyl-α-L-rhamnopyranosid
p-Aminophenyl-β-D-galactopyranosid
p-Aminophenyl-2-O-methyl-β-D-galactopyranosid
p-Amlnophenyl-3-O-methyl-β-D-galactopyranosid
p-Aminophenyl-4-O-methyl-β-D-galactopyranosid
p-Aminophenyl-6-O-methyl-β-D-galactopyranosid
p-Aminophenyl-2,3-di-O-methyl-β-D-galactopyranosid
p-Aminophenyl-2,4-di-O-methyl-β-D-galactopyranosid
p-Aminophenyl-2,6-di-O-methyl-β-D-galactopyranosid
p-Aminophenyl-3,4-di-O-methyl-β-D-galactopyranosid, Acetat
p-Aminophenyl-3,6-di-O-methyl-β-D-galactopyranosid
p-Aminophenyl-4,6-di-O-methyl-β-D-galactopyranosid
p-Aminophenyl-2,3,4-tri-O-methyl-β-D-galactopyranosid
p-Aminophenyl-2,3,6-tri-O-methyl-β-D-galactopyranosid
p-Arninophenyl-2,4,6-tri-O-methyl-β-D-galactopyranosid
p-Aminophenyl-3,4,6-tri-O-methyl-β-D-galactopyranosid
p-Aminophenyl-3-desoxy-β-D-galactopyranosid
p-Aminophenyl-3,4-didesoxy-β-D-galactopyranosid
p-Aminophenyl-6-O-acetyl-β-D-galactopyranosid
p-Aminophenyl-3-O-methoxycarbonylmethyl-β-D-galactopyranosid
p-Aminophenyl-3-O-carboxymethyl-β-D-galactopyranosid, Natriumsalz
p-Aminophenyl-3-O-carbamoylmethyl-β-D-galactopyranosid
p-Aminophenyl-3-O-(N-methyl-carbamoylmethyl)-β-D-galactopyranosid
p-Aminophenyl-α-D-mannopyranosid
p-Aminophenyl-3-O-methyl-α-D-mannopyranosid
p-Aminophenyl-2,3-di-O-methyl-α-D-mannopyranosid
p-Aminophenyl-3-O-methoxycarbonylmethyl-α-D-mannopyranosid
p-Aminophenyl-3-O-carboxymethyl-α-D-mannopyranosid
p-Aminophenyl-3-O-carbamoylmethyl-α-D-mannopyranosid
p-Aminophenyl-4-O-(β-D-galactopyranosyl)-β-D-glucopyranosid
p-Aminophenyl-4-O-(3'-sulfat-β-D-galactopyranosyl)-β-D-glucopyranosid,Natriumsalz
p-Aminophenyl-4-O-(3'-O-methyl-β-D-galactopyranosyl)-β-D-glucopyranosid
p-Aminophenyl-2-O-methyl-4-O-(3'-O-methyl-β-D-galactopyranosyl)-β-D-glucopyranosid
p-Aminophenyl-4-O-(3',4'-di-O-methyl-β-D-galactopyranosyl)-β-D-glucopyranosid

Bereits in EP 501 250 wurde die Synthese der folgenden Kohlenhydratbausteine beschrieben:
Carboxymethyl-β-L-fucopyranosid
5-Carboxypentyl-β-L-fucopyranosid

Die Verknüpfung dieser beiden Bausteine mit Peptidkonjugaten kann in der in EP 501 250 beschriebenen Weise erfolgen.

### Glycokonjugate

### Beispiel 1.1:

### 20(S)-7-Ethyl-20-O-{N^{α},N^{ε}-bis-[O-(3-O-methyl-β-L-fucopyranosyl)-4-hydroxyphenylamino-thiocarbonyl]-lysyl-alanyl}-camptothecin:

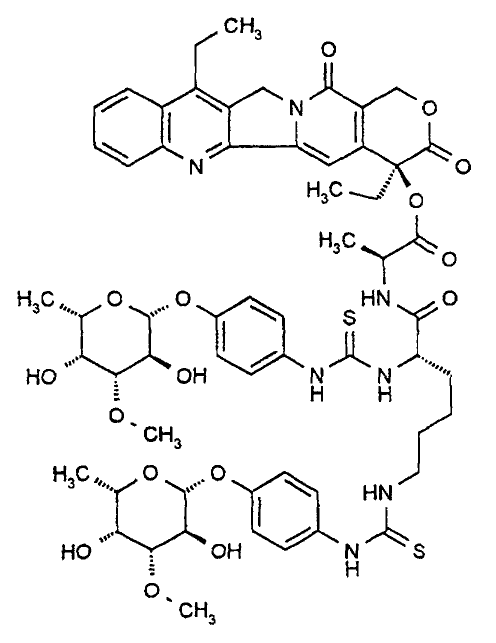

### 1.1.a) 20(S)-7-Ethyl-20-O-[N-(tert-butoxycarbonyl)-alanyl]-camptothecin:

Eine Lösung von 1,88 g (5,0 mmol) 20(S)-7-Ethyl-camptothecin (S. Sawada et al, Chem.Pharm.Bull., 39 (1991) 1446-1454) in 100 ml absolutem Dimethylformamid wird unter Rühren mit 2,15 g (10,0 mmol) N-(tert-Butoxycarbonyl)-alanin-N-carbonsäureanhydrid sowie 150 mg (1,2 mmol) 4-(N,N-Dimethylamino)-pyridin versetzt. Nach 3 h bei Raumtemperatur setzt man weitere 2,15 g (10,0 mmol) N-(tert-Butoxycarbonyl)-alanin-N-carbonsäureanhydrid und 150 mg (1,2 mmol) 4-(N,N-Dimethylamino)-pyridin zu und rührt über Nacht bei Raumtemperatur. Anschließend wird im Vakuum eingeengt und der Rückstand durch Flash-chromatographie [Petrolether/Ethylacetat 2:1 -> 1.1 -> Ethylacetat] gereinigt. Man erhält farblose Kristalle (2,02 g, 73,8 %); DC [Ethylacetat] R_{f} = 0,56; Schmp = 206-212°C; FAB-MS: m/z = 548 (M+H⁺).

### 1.1.b) 20(S)-20-O-Alanyl-7-ethyl-camptothecin, Trifluoracetat:

Eine Lösung von Verbindung 1.1.a (1,81 g, 3,3 mmol) in einer Mischung aus 70 ml Dichlormethan und 7 ml wasserfreier Trifluoressigsäure wird für 90 min. bei Raumtemperatur gerührt. Nach Einengen im Vakuum auf ein kleines Volumen wird das Produkt mit Diethylether ausgefällt und gründlich mit Diethylether gewaschen. Das Produkt fällt in hellgelben Kristallen (1,34 g, 72,3 %) an; DC [Ethylacetat]: R_{f} = 0,05; Schmp. = 242°C (Zers.).

### 1.1.c) 20(S)-7-Ethyl-20-O-[N^{α},N^{ε}-di-(tert-butoxycarbonyl)-lysyl-alanyl]-camptothecin:

1,57 g (4,55 mmol) N,N-Di-(tert-butoxycarbonyl)-lysin und 923 mg (6,83 mmol) 1-Hydroxy-1H-benzotriazol Hydrat werden in 35 ml Dimethylformamid gelost. Nach Zugabe von 1,09 g (5,7 mmol) N-Ethyl-N'-(dimethylaminopropyl)-carbodiimid Hydrochlorid und 990 µl (5,7 mmol) Ethyl-diisopropylamin ruhrt man für 30 min. bei Raumtemperatur. Anschließend setzt man eine Lösung aus Verbindung 1.1.b (1,3 g, 2,32 mmol) in 35 ml Dimethylformamid und 408 µl (2,32 mmol) Ethyl-diisopropylamin zu und rührt den Ansatz für weitere 16 h bei Raumtemperatur. Nach Einengen im Vakuum und Reinigung durch Flashchromatographie [Petrolether/Ethylacetat 2:1 -> 1:1 -> Ethylacetat] erhält man hellgelbe Kristalle (1,38 g, 75,3 %); DC [Ethylacetat]: R_{f} = 0,53; Schmp. = 125°C (Zers.).

### 1.1.d) 20(S)-7-Ethyl-20-O-(lysyl-alanyl)-camptothecin, Di-Trifluoracetat:

Eine Suspension der obigen Verbindung (1,18 g, 1,5 mmol) in Dichlormethan (50 ml) wird mit wasserfreier Trifluoressigsäure (5 ml) versetzt und die resultierende Lösung für 1 h bei Raumtemperatur gerührt. Nach Einengen auf ein kleines Volumen im Vakuum wird das Produkt durch Zugabe von Diethylether ausgefällt. Der Niederschlag wird abfiltriert und aus Ethylacetat umkristallisiert. Man erhält gelbe Kristalle (862 mg, 71,5 %); DC [Ethylacetat]: R_{f} = 0,05; Schmp. = 137°C (Zers).

### 1.1) 20(S)-7-Ethyl-20-O-{N^{α},N^{ε}-bis-[O-(3-O-methyl-β-L-fucopyranosyl)-4-hydroxy-phenylamino-thiocarbonyl]-lysyl-alanyl}-camptothecin:

Eine Lösung von 269,4 mg (1,0 mmol) p-Aminophenyl-3-O-methyl-β-L-fucopyranosid (Beispiel I.1) in Dioxan/Wasser 1:1 (10 ml) wird unter Rühren mit Thiophosgen (150 µl, 2,0 mmol) versetzt. Nach 10 min. versetzt man mit 4 Äquivalenten Ethyldiisopropylamin, engt anschließend im Vakuum ein und trocknet den Rückstand für 1 h im Ölpumpenvakuum. Das erhaltene Isothiocyanat wird in 50 ml absolutem Dimethylformamid gelöst und mit 361,7 mg (0,45 mmol) von Verbindung 1.1.d sowie 317 µl (1,8 mmol) Ethyl-diisopropylamin versetzt. Man rührt für 16 h bei Raumtemperatur, engt dann im Vakuum ein und reinigt durch Flash-Chromatographie [Acetonitril/Wasser 15:1 -> 10.1 -> 5:1]. Das Produkt wird aus Dichlormethan/Methanol mit Diethylether umgefällt. Man erhält 203,3 mg (37,7 %) des Zielproduktes als gelbe Kristalle; Schmp. = 196-198°C (Zers.).

### Beispiel 1.2:

### 20(S)-7-Ethyl-20-O-{N^{α},N^{ε}-bis-[O-(3-O-carboxymethyl-β-L-fucopyranosyl)-4-hydroxy-phenylamino-thiocarbonyl]-lysyl-alanyl}-camptothecin, Di-Natriumsalz:

Das Glycokonjugat wird in Analogie zu Beispiel 1.1 aus 361,7 mg (0,45 mmol) Peptidkonjugat 1.1.d und 313,3 mg (1,0 mmol) p-Aminophenyl-3-O-carboxymethyl-β-L-fucopyranosid (Beispiel 1.2) hergestellt.

Reinigung durch Flashchromatographie [Acetonitril/Wasser 10:1 -> 5-1]. Uberführung in das Di-Natriumsalz mit 2 Äquivalenten einer 0,1N Natronlauge. Man erhält einen gelben, amorphen Feststoff (162,8 mg, 27,2 %); DC [Acetonitril/Wasser 5 1] R_{f} = 0,09.

### Beispiel 1.3:

### 20(S)-7-Ethyl-20-O-{N^{α}-[O-(3-O-carboxymethyl-β-L-fucopyranosyl)-4-hydroxyphenylamino-thiocarbonyl]-lysyl-alanyl}-camptothecin, Hydrochlorid:

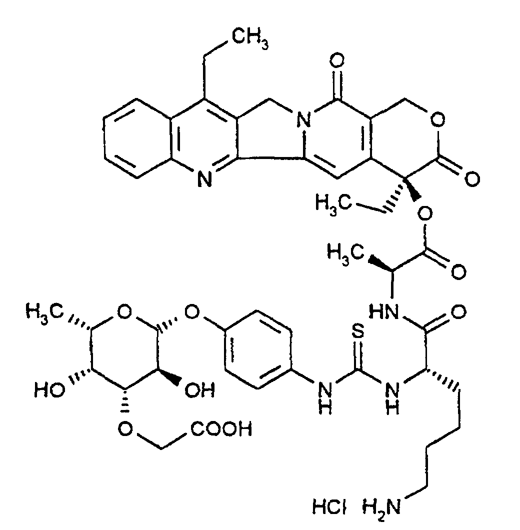

### 1.3.a) 20(S)-7-Ethyl-20-O-[N^{ε}-(fluorenyl-9-methoxycarbonyl)-lysyl-alanyl]-camptothecin, Trifluoracetat:

Das Konjugat aus Beispiel 1.1.b wird in Analogie zur Vorschrift von Beispiel 1.1.c. mit N^{α}-(tert-Butoxycarbonyl)-N^{ε}-(fluorenyl-9-methoxycarbonyl)-lysin verknüpft und anschließend an der α-Aminofunktion gemäß Beispiel I.1.d. deblockiert.

### 1.3.b) 20(S)-7-Ethyl-20-O-{N^{α}-[O-(3-O-carboxymethyl-β-L-fucopyranosyl)-4-hydroxy-phenylamino-thiocarbonyl]-N^{ε}-[fluorenyl-9-methoxycarbonyl]-lysyl-alanyl}-camptothecin:

Die Verbindung aus Beispiel 1.3.a wird in Analogie zu Beispiel 1.1 mit 1,2 Äquivalenten des Kohlenhydratderivats aus Beispiel I.2 umgesetzt.

### 1.3) 20(S)-7-Ethyl-20-O-{N^{α}-[O-(3-O-carboxymethyl-β-L-fucopyranosyl)-4-hydroxy-phenylamino-thiocarbonyl]-lysyl-alanyl}-camptothecin, Hydrochlorid:

Das Konjugat 1.3.b wird mit Piperidin in DMF deblockiert. Nach 30min. engt man ein und digeriert den Rückstand zweimal mit Dichlormethan. Dann wird in DMF aufgenommen und mit Methanol/Ether gefällt. Das Produkt wird abgesaugt, mit Ether gewaschen und dann aus Dioxan/Wasser lyophilisiert. Anschließend nimmt man in Wasser auf, versetzt mit 1 Äquivalent einer 0,01N HCl-Lösung und lyophilisiert erneut.

### Beispiel 1.4:

### 20(S)-7-Ethyl-20-O-{N^{α}-[O-(3-O-carboxymethyl-β-L-fucopyranosyl)-4-hydroxyphenylamino-thiocarbonyl]-lysyl-valyl}-camptothecin, Hydrochlorid

Die Verbindung wird analog zu 1.3 hergestellt.

### Beispiel 1.5

### 7-Ethyl-20-O-{N^{α}-[O-(3-O-methyl-β-L-fucopyranosyl)-4-hydroxyl-phenylamino-thiocarbonyl]-lysyl-valyl}-camptothecin, Hydrochlorid:

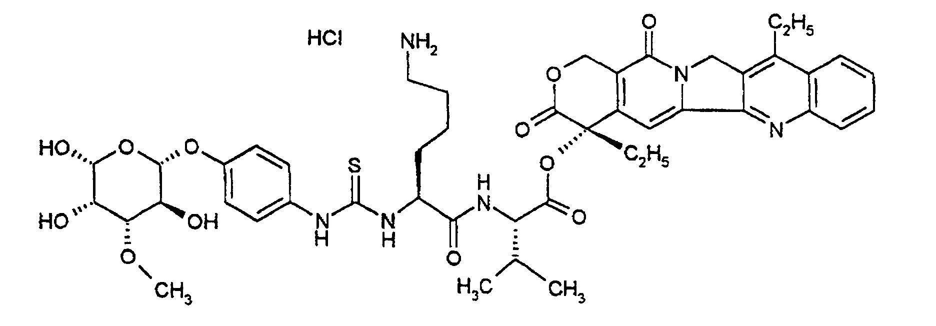

### 1.5.a) 20-O-[N-(tert-Butoxycarbonyl)-valyl)-7-ethyl-camptothecin:

Die Verbindung wird unter Verwendung des in 1.1.a beschriebenen Verfahrens aus 1,88 g (5,0 mmol) 20(S)-7-Ethyl-camptothecin (S. Sawada et al., Chem Pharm. Bull. 39 (1991) 1446-1454) und 2,43 g (10,0 mmol) N-(tert-Butoxycarbonyl)valin-N-carbonsaureanhydrid hergestellt. Man erhält 1,46 g (51 % an beigen Kristallen [DC (Acetonitril): R_{f} = 0,86; Schmp. = 224-227°C (Zers.), FAB-MS: m/z = 576 (M+H⁺).

### 1.5.b) 7-Ethyl-20-O-valyl-camptothecin, Trifluoracetat:

Aus Verbindung 1.5.a (1,44 g, 2,5 mmol) wird wie unter 1.1.b beschrieben die N-(tert-Butoxycarbonyl)-Gruppe abgespalten. Man erhält 626 mg (43 %) an gelben Kristallen [DC (Acetonitril): R_{f} = 0,45; Schmp. = 160°C Zers.)].

### 1.5.c) 20-O-[N^{α}-(tert-Butoxycarbonyl)-N^{ε}-(fluorenyl-9-methoxycarbonyl)-lysyl-valyl]-7-ethyl-camptothecin:

In Analogie zu 1.1.c werden 797 mg (1,7 mmol) N^{α}-(tert-Butoxycarbonyl)-N^{ε}-(fluorenyl-9-methoxycarbonyl)-lysin mit Verbindung 1.5.b (590 mg, 1,0 mmol) umgesetzt. Nach Einengen im Vakuum und Reinigung durch Flashchromatographie [Petrolether/Ethylacetat 1:2] erhält man beige Kristalle. Ausb.: 287 mg (31 %) [DC (Ethylacetat): R_{f} = 0,50; Schmp = 172°C (Zers.)].

### 1.5.d) 7-Ethyl-20-O-[N^{ε}-(fluorenyl-9-methoxycarbonyl)-lysyl-valyl]-camptothecin, Trifluoracetat:

Obige Verbindung (277,8 mg, 0,3 mmol) wird wie beschrieben mit Trifluoressigsäure in Dichlormethan entschützt. Man erhält 209 mg (74 %) an gelben Kristallen [DC (Ethylacetat): R_{f} = 0,06; Schmp. = 199°C (Zers.)].

### 1.5.e) 20(S)-7-Ethyl-20-O-{N^{α}-(O-(3-O-methyl-β-L-fucopyranosyl)-4-hydroxyphenylamino-thiocarbonyl]-N^{ε}-{fluorenyl-9-methoxy-carbonyl]-lysylvalyl}-camptothecin:

Die Verbindung aus Beispiel 1.5.d wird in Analogie zu Beispiel 1.1 mit 1,2 Äquivalenten des Kohlenhydratderivats aus Beispiel I.1 umgesetzt und das Produkt durch Umfällen aus Dichlormethan/Methanol 1:1 mit Diethylether gereinigt. Man erhält in 35 %-iger Ausbeute beige Kristalle [DC (Acetonitril/Ethylacetat 1:1): R_{f} = 0,68].

### 1.5) 20(S)-7-Ethyl-20-O-{N^{α}-[O-(3-O-methyl-β-L-fucopyranosyl)-4-hydroxyphenylamino-thiocarbonyl-]-lysyl-valyl}-camptothecin, Hydrochlorid:

Das Konjugat 1.5.e wird mit Piperidin in DMF deblockiert. Nach 3 h engt man ein und fällt den Rückstand zweimal aus Dichlormethan/Methanol 1:1 mit Diethylether um. Anschließend nimmt man in Wasser auf, versetzt mit 1 Äquivalent einer 0,01N HCl-Lösung und lyophilisiert.

### Beispiel 2.1:

### 20(R,S)-10,11-Methylendioxy-20-O-{N^{α},N^{ε}-bis-[O-(3-O-methyl-β-L-fucopyranosyl)-4-hydroxy-phenylamino-thiocarbonyl]-lysyl-alanyl}-camptothecin

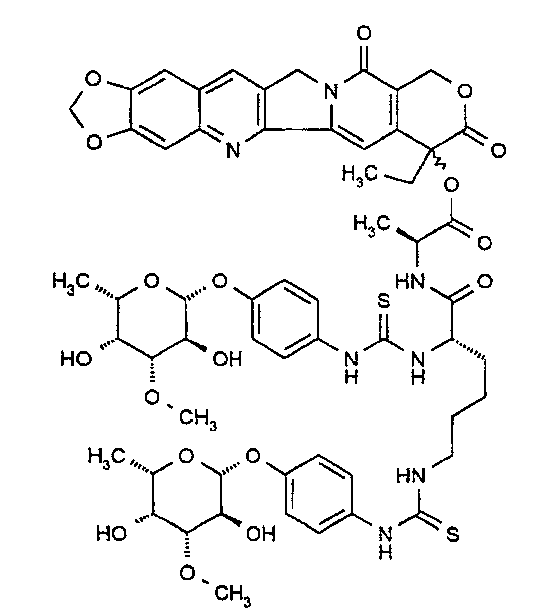

### 2.1.a) 20(R/S)-10,11-Methylendioxy-camptothecin:

Dieses Camptothecin-Derivat wird nach Wall et al. (J.Med.Chem., 29 (1986), 2358) aus dem racemischen Tricyclus hergestellt.

### 2.1.b) 20(R,S)-10,11-Methylendioxy-20-O-alanyl-camptothecin, Trifluoracetat:

Eine Lösung von 300 mg (0,765 mmol) 20(R/S)-10,11-Methylendioxy-camptothecin in 30 ml absolutem Dimethylformamid wird unter Rühren mit 330 mg (1,53 mmol) N-(tert-Butoxycarbonyl)-alanin-N-carbonsäureanhydrid sowie 30 mg 4-(N,N-Dimethylamino)-pyridin versetzt. Nach 24 h Rühren bei Raumtemperatur setzt man jeweils zwei weitere Äquivalente N-(tert-Butoxycarbonyl)-alanin-N-carbonsäureanhydrid und 30 mg 4-(N,N-Dimethylamino)-pyridin zu. Nach Abschluß der Reaktion wird im Vakuum eingeengt. Nach Fällen aus Dichlormethan mit Ether erhält man die geschützte Zwischenstufe in 80 %iger Ausbeute. [DC: Dichlormethan/Methanol 95:5 R_{f} = 0,38]. Man nimmt in 10 ml Dichlormethan auf und setzt bei 0°C 2 ml wasserfreie Trifluoressigsäure zu. Nach 15 min. wird eingeengt und aus Dichlormethan/Methanol mit Ether gefällt.
Ausb.: 97 % [FAB-MS: m/z = 464 = M+H]

### 2.1) 20(R,S)-10,11-Methylendioxy-20-O-{N^{α},N^{ε}-bis-[O-(3-O-methyl-β-L-fucopyranosyl)-4-hydroxy-phenylamino-thiocarbonyl]-lysyl-alanyl}-camptothecin

Das Glycokonjugat wird in Analogie zu den Vorschriften zu 1.1.c, 1.1.d und 1.1 aus dem Alanyl-Konjugat in Beispiel 2.1.b hergestellt.

Ausb . 87 % [DC: Acetonitril/Wasser 10:1 R_{f} = 0,33] [FAB-MS: m/z = 1214 = M+H].

### Beispiel 2.2:

### 20(S)-10,11-Methylendioxy-20-O-{N^{α},N^{ε}-bis-[O-(3-O-methyl-β-L-fucopyranosyl)-4-hydroxy-phenylamino-thiocarbonyl]-lysyl-alanyl}-camptothecin

### 2.2.a) 20(S)-10,11-Methylendioxy-camptothecin:

Dieses Camptothecin-Derivat wird nach Wall et al. (J Med.Chem., 29 (1986), 2358) aus dem S-konfigurierten, enantiomerenreinen Tricyclus, der durch Racematspaltung gewonnen werden kann, hergestellt

### 2.2) 20(S)-10,11-Methylendioxy-20-O-{N^{α},N^{ε}-bis-[O-(3-O-methyl-β-L-fucopyranosyl)-4-hydroxy-phenylamino-thiocarbonyl]-lysyl-alanyl}-camptothecin

Darstellung in Analogie zu Beispiel 2.1.

### Beispiel 2.3:

### 20(S)-10,11-Methylendioxy-20-O-{N^{α}-[O-(3-O-carboxymethyl-β-L-fucopyranosyl)-4-hydroxy-phenylamino-thiocarbonyl]-lysyl-alanyl}-camptothecin, Hydrochlorid

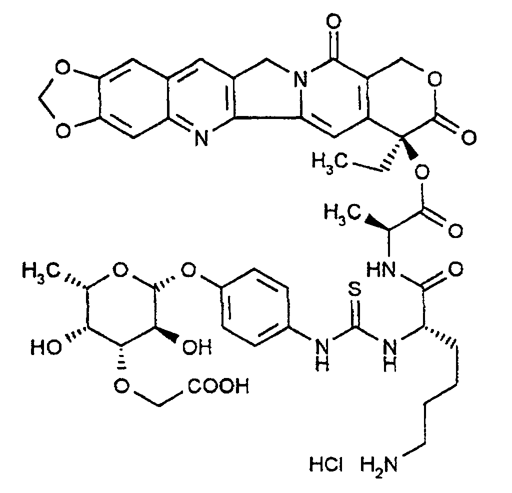

### 2.3.a) 20(S)-10,11-Methylendioxy-20-O-alanyl-camptothecin, Trifluoracetat:

Dieses Konjugat wird aus der Verbindung 2.2.a in Analogie zu 2.1.b hergestellt. Ausb.: 72% über 2 Stufen [FAB-MS: m/z = 464 = M+H]

### 2.3.b) 20(S)-10,11-Methylendioxy-20-O-{N^{ε}-[fluorenyl-9-methoxycarbonyl]-lysyl-alanyl}-camptothecin, Trifluoracetat:

Das Konjugat aus Beispiel 2.3.a wird in Analogie zur Vorschrift von Beispiel 1.1.c. mit N^{α}-(tert-Butoxycarbonyl)-N^{ε}-(fluorenyl-9-methoxycarbonyl)-lysin verknüpft und anschließend an der α-Aminofunktion durch Einwirkung von Trifluoressigsäure deblockiert. Ausb.: 52 % über 2 Stufen.

### 2.3.c) 20(S)-10,11-Methylendioxy-20-O-{N^{α}-[O-(3-O-carboxymethyl-β-L-fucopyranosyl)-4-hydroxy-phenylamino-thiocarbonyl]-N^{ε}-[fluorenyl-9-methoxycarbonyl]-lysyl-alanyl}-camptothecin:

Die Verbindung aus Beispiel 2.3.b wird in Analogie zu Beispiel 1.1 mit 1,2 Äquivalenten des Kohlenhydratderivats aus Beispiel 1.2 umgesetzt. Das Rohprodukt wird durch Verrühren mit Wasser und anschließende Fällung aus Dichlormethan/Methanol mit Ether gereinigt.
Ausb.: 88 % [DC: Dichlormethan/Methanol/Ammoniak 17 %ig 15.4:0,5 R_{f} = 0,27]

### 2.3) 20(S)-10,11-Methylendioxy-20-O-{N^{α}-[O-(3-O-carboxymethyl-β-L-fucopyranosyl)-4-hydroxy-phenylamino-thiocarbonyl]-lysyl-alanyl}-camptothecin, Hydrochlorid

Das Konjugat 2.3.c wird mit Piperidin in DMF deblockiert. Nach 20min. engt man ein und digeriert den Rückstand zweimal mit Dichlormethan. Das Produkt wird abgesaugt, noch einmal mit Ether gewaschen und dann aus Dioxan/Wasser lyophilisiert Anschließend nimmt man in Dioxan/Wasser auf, versetzt mit 1 Äquivalent einer 0,1N HCl-Lösung und lyophilisiert erneut. Ausb.: quant. [DC: Acetonitril/Wasser/Eisessig 5:1:0,2 R_{f} = 0,26] [FAB-MS: m/z = 947 = M+H]

### Beispiel 2.4:

### 20(S)-10,11-Methylendioxy-20-O-{N^{α}-[O-(3-O-Methyl-β-L-fucopyranosyl)-4-hydroxy-phenylamino-thiocarbonyl]-lysyl-leucyl}-camptothecin, hydrochlorid

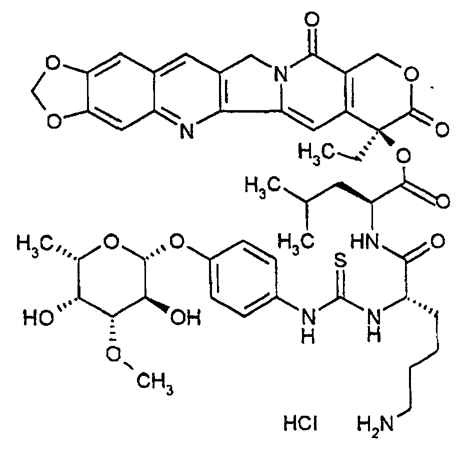

### 2.4.a) 20(S)-10,11-Methylendioxy-20-O-leucyl-camptothecin, Trifluoracetat

Ausgehend von 20(S)-10,11-Methylendioxy-camptothecin (Beispiel 2.2.a) wird mit N-tert-Butoxycarbonyl)-leucin-N-carbonsäureanhydrid in Analogie zu Beispiel 2.1.b die Zielverbindung hergestellt.

### 2.4.b) 20(S)-10,11-Methylendioxy-20-O-{N^{ε}-[fluorenyl-9-methoxycarbonyl]-lysyl-leucyl}-camptothecin, Trifluoracetat:

Das Konjugat aus Beispiel 2.4.a wird in Analogie zur Vorschrift von Beispiel 1.1.c mit N^{α}-(tert-Butoxycarbonyl)-N^{ε}-(fluorenyl-9-methoxycarbonyl)-lysin verknüpft und anschließend an der α-Aminofuntion durch Einwirkung von Trifluoressigsaure deblocliert. Ausb.: 69 % über 2 Stufen.

### 2.4.c) 20(S)-10,11-Methylendioxy-20-O-{N^{α}-[O-(3-O-methyl-β-L-fucopyranosyl)-4-hydroxy-phenylamino-thiocarbonyl]-N^{ε}-[fluorenyl-9-methoxycarbonyl]-lysyl-leucyl}-camptothecin:

Die Verbindung aus Beispiel 2.4.b wird in Analogie zu Beispiel 1.1 mit 1,2 Äquivalenten des Kohlenhydratderivats aus Beispiel I.1 umgesetzt. Das Rohprodukt wird durch Fällung aus Dichlormethan/Methanol mit Ether gereinigt.
Ausb.: 95 % [DC. Acetonitril/Wasser 20:1 R_{f} = 0,48].

### 2.4) 20(S)-10,11-Methylendioxy-20-O-{N^{α}-[O-(3-O-methyl-β-L-fucopyranosyl)-4-hydroxy-phenylamino-thiocarbonyl]-lysyl-leucyl}-camptothecin, Hydrochlorid.

Das Konjugat 2.4.c wird mit Piperidin in DMF deblockiert. Nach 20 min. engt man ein und destilliert zweimal mit Dichlormethan nach. Das Produkt wird aus Dichlormethan/Methanol mit Ether gefällt Anschließend nimmt man in Wasser auf, versetzt mit 1 Äquivalent einer 0,1N HCl-Lösung und lyophilisiert. Ausb.: 75 % [DC: Acetonitril/Wasser/Eisessig 5:1:0,2 R_{f} = 0,47]

### Beispiel 3.1:

### 20(S)-9-Amino-20-O-{N^{α},N^{ε}-bis-[O-(3-O-methyl-β-L-fucopyranosyl)-4-hydroxy-phenylamino-thiocarbonyl]-lysyl-alanyl}-camptothecin

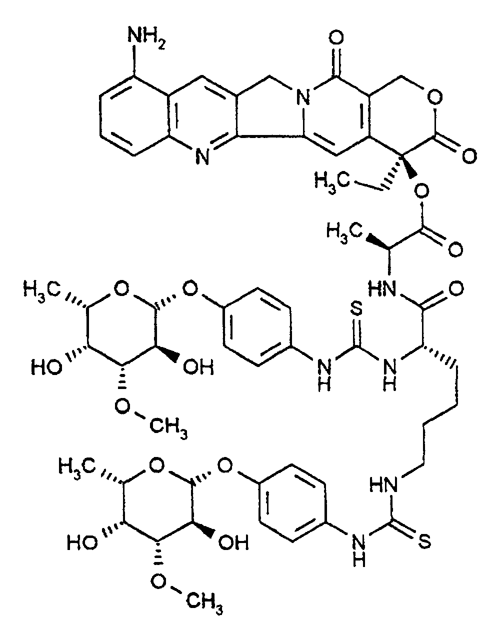

### 3.1.a) 20(S)-9-Nitro-camptothecin:

Dieses Derivat wird nach der Vorschrift von Wani et al. (J.Med.Chem. 29 (1986), 2358) hergestellt.

### 3.1.b) 20(S)-9-Nitro-20-O-alanyl-camptothecin, Trifluoracetat:

516mg (1,31mmol) der Verbindung aus Beispiel 3.1.a werden in 55 ml absolutem Dimethylformamid unter Rühren mit 846 mg (3,9 mmol) N-(tert-Butoxycarbonyl)-alanin-N-carbonsäureanhydrid sowie 55 mg 4-(N,N-Dimethylamino)-pyridin versetzt. Nach 4 h wird im Vakuum eingeengt und mit Dichlormethan/Essigester 2:1 an Kieselgel chromatographiert. Nach erneutem Einsatz des zurückgewonnen Edukts unter gleichen Bedingungen erhält man die geschützte Zwischenstufe in 54 %iger Ausbeute. [DC: Dichlormethan/Methanol 95:5 R_{f} = 0,5]. Man nimmt in 40 ml Dichlormethan auf und setzt bei 0°C 4 ml wasserfreie Trifluoressigsäure zu. Nach 15 min. wird eingeengt und durch Verrühren mit Ether gereinigt Ausb.. 80 %

### 3.1.c) 20(S)-9-Amino-20-O-alanyl-camptothecin, Trifluoracetat:

320 mg (0,55 mmol) der Verbindung aus 3.1.b werden in 30 ml Ethanol aufgenommen und 15 min. über 10 mg Platindioxid hydriert. Der Katalysator wird abfiltriert, die Lösung eingeengt und das Produkt nach Fällung aus Dichlormethan mit Methanol in die nächste Stufe eingesetzt.

### 3.1.d) 20(S)-9-Amino-20-O-(lysyl-alanyl)-camptothecin, Di-Trifluoracetat:

31 mg (0,09mmol) N,N-Di-(tert-butoxycarbonyl)-lysin werden in 5 ml Dimethylformamid mit 15 mg (0,135mmol) N-Hydroxy-succinimid und 22 mg (0,11 mmol) Dicyclohexyl-carbodiimid für 2 h voraktiviert. Anschließend setzt man Verbindung 3.1.c (60 mg, 0,09 mmol) und 31µl Ethyl-diisopropylamin zu und rührt den Ansatz für weitere 16 h bei Raumtemperatur. Nach Einengen im Vakuum und Reinigung durch Flashchromatographie [Toluol/Ethanol 8:1] erhält man die Zwischenstufe in 47 %iger Ausbeute. [DC: Dichlormethan/Methanol 9:1 R_{f} = 0,31] 30 mg (0,039 mmol) werden in Dichlormethan (10 ml) mit wasserfreier Trifluoressigsäure (2 ml) versetzt und die resultierende Lösung für 30 min. bei Raumtemperatur gerührt Nach Einengen wird das Produkt aus Dichlormethan/Methanol mit Ether ausgefällt (20 mg, 57 %). [DC: Acetonitril/Wasser/Eisessig 10:5:3 R_{f} = 0,44]

### 3.1) 20(S)-9-Amino-20-O-{N^{α},N^{ε}-bis-[O-(3-O-methyl-β-L-fucopyranosyl)-4-hydroxy-phenylamino-thiocarbonyl]-lysyl-alanyl}-camptothecin

Das Glycokonjugat wird aus den Verbindungen in 3.1.d und I.1 in Analogie zu Beispiel 1.1 hergestellt. Ausb.: 58 % [DC: Acetonitril/Wasser/Eisessig 5.1:0,2 R_{f} = 0,6].

### Beispiel 4.1:

### 20(S)-7-{N^{α},N^{ε}-bis-[O-(3-O-Methyl-β-L-fucopyranosyl)-4-hydroxyphenylamino-thiocarbonyl]-lysyl-alanyloxymethyl}-20-O-{N^{α},N^{ε}-bis-[O-(3-O-methyl-β-L-fucopyranosyl)-4-hydroxy-phenylamino-thiocarbonyl]-lysyl-alanyl}-camptothecin

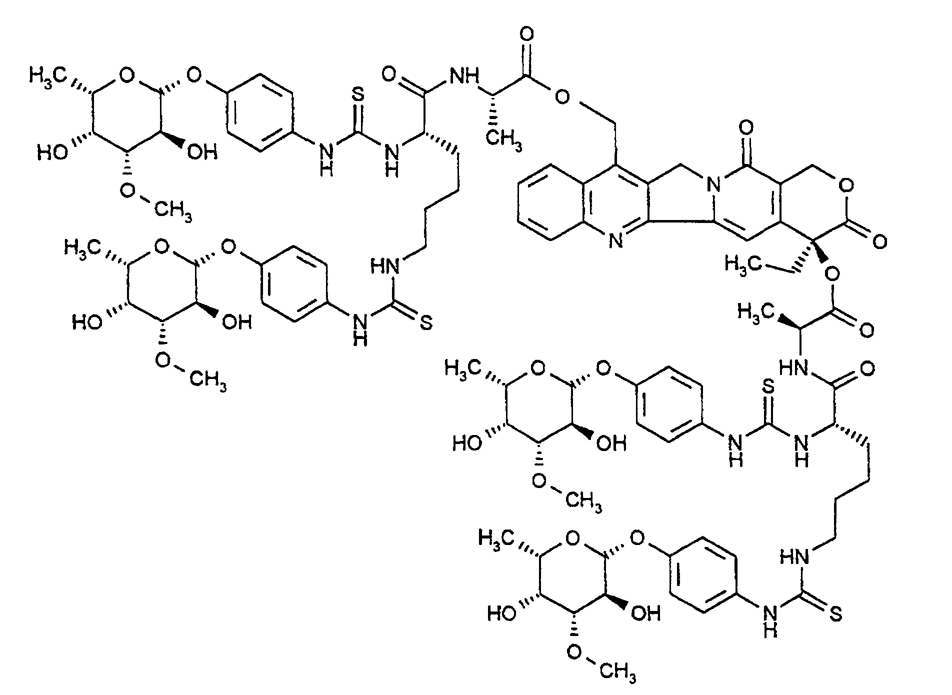

### 4.1.a) 20(S)-7-Hydroxymethyl-camptothecin:

Diese Verbindung wird nach der Vorschrift von Miyasaka et al. (Chem. Pharm. Bull. 39 (1991) 2574) hergestellt.

### 4.1.b) 20(S)-7-(Alanyloxymethyl)-20-O-alanyl-camptothecin, Di-Trifluoracetat:

1 g (2,64 mmol) 20(S)-7-Hydroxymethyl-camptothecin werden in 100 ml DMF gelöst und dann mit 100 mg 4-N,N-Dimethylaminopyridin und 1,5 Äquivalenten N-(tert-Butoxycarbonyl)-L-alanin-N-carboxy-anhydrid versetzt und die Suspension 16 h bei Raumtemperatur gerührt. Um vollständige Doppelacylierung zu erreichen, setzt man erneut 100 mg 4-N,N-Dimethylaminopyridin und 1,5 Aquivalente N-(tert-Butoxycarbonyl)-alanin-N-carboxy-anhydrid zu und beläßt weitere 16 h bei Raumtemperatur. Man engt ein und reinigt durch Flash-Chromatographie an Ethylacetat/Petrolether 1:1. Das gereinigte Material wird in 30 ml Dichlormethan aufgenommen und bei 0°C mit 5 ml Trifluoressigsäure versetzt. Nach 30 min Rühren wird eingeengt und das aminodeblockierte Produkt aus Dichlormethan/Ether gefällt. Anschließend wird aus Dioxan/Wasser lyophilisiert. [DC: Acetonitril/-Wasser/Eisessig 10:5:5 R_{f} = 0,17] [FAB-MS: m/z = 777 = M+H].

### 4.1) 20(S)-7-{N^{α},N^{ε}-bis-[O-(3-O-methyl-β-L-fucopyranosyl)-4-hydroxyphenylamino-thiocarbonyl]-lysyl-alanyloxymethyl}-20-O-{N^{α},N^{ε}-bis-[O-(3-O-methyl-β-L-fucopyranosyl)-4-hydroxy-phenylamino-thiocarbonyl]-lysyl-alanyl}-camptothecin

Das Konjugat wird aus Beispiel 4.1.b in Analogie zu den Beispielen 1.1.c, 1.1.d und 1.1 hergestellt, auf allen Stufen jedoch mit der doppelten Menge der jeweiligen Reaktanden. [DC: Acetonitril/Wasser 10:1 R_{f} = 0,2] [MALDI-MS: m/z = 2047 = M(+3)+Na].

### Beispiel 5.1:

### 20(S)-7-[4-(Methylpiperazino)methyl]-10,11-(ethylendioxy)-20-O-{N^{α}-[O-(3-O-methyl-β-L-fucopyranosyl)-4-hydroxy-phenylamino-thiocarbonyl]-lysyl-leucyl}-camptothecin, Hydrochlorid

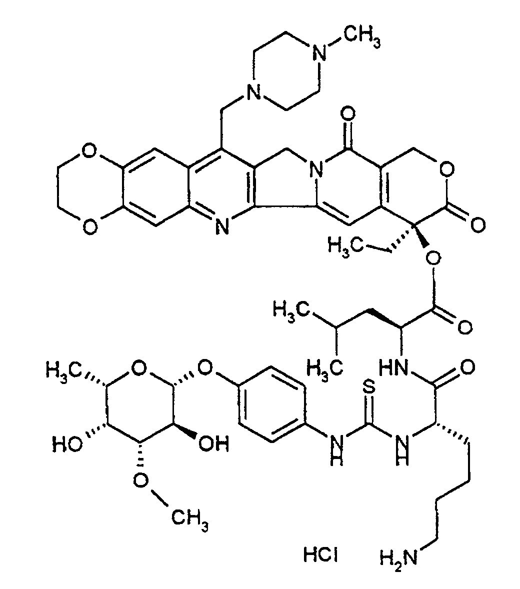

### 5.1.a) 20(S)-7-[Methylpiperazino)methyl]-10,11-(ethylendioxy)-camptothecin, Trifluoracetat

Dieses Derivat wird nach der Vorschrift von Luzzio et al. (J.Med.Chem. 38 (1995), 396) hergestellt.

### 5.1.b) 20(S)-7-[-4-lMethylpiperazino)methyl]-10,11-(ethylendioxy)-20-O-leucylcamptothecin, Tri-Trifluoracetat

Ausgehend von der Verbindung aus Beispiel 5.1.a) wird mit N-[tert-Butoxycarbonyl)-leucin-N-carbonsäureanhydrid (10 Äquivalente) in Analogie zu Beispiel 2.1.b die Zielverbindung hergestellt. Die Reinigung der Boc-geschützten Zwischenstufe erfolgt durch Flash-Chromatographie [Dichlormethan/Methanol/Ammoniak 17%ig 15:1:0,1]. Ausb.: 62% über 2 Stufen [ESI-MS: m/z = 632 = M+H; m/z = 654 = M+Na].

### 5.1.c) 20(S)-7-[4-Methylpiperazino)methyl-10,11-(ethylendioxy)-20-O-{N^{ε}-[fluorenyl-9-methoxycarbonyl]-lysyl-leucyl}-camptothecin, Tri-Trifluoracetat:

Das Konjugat aus Beispiel 5.1.b wird in Analogie zur Vorschrift von Beispiel 1.1.c mit N^{α}-(tert-Butoxycarbonyl)-N^{ε}-(fluorenyl)-9-methoxycarbonyl)-lysin verknüpft und anschließend an der α-Aminofunktion durch Einwirkung von Trifluoressigsäure deblockiert. Die Reinigung der Boc-geschützten Zwischenstufe erfolgt durch Flash-Chromatographie [Dichlormethan/Methanol 10:1] Ausb.: 53 % über 2 Stufen. [DC. Acetonitril/Wasser/Eisessig 5:1:0,2 R_{f} = 0,13] [FAB-MS: m/z = 982 = M+H]

### 5.1.d) 20(S)-7-[4-(Methylpiperazino)methyl]-10,11-(ethylendioxy)-20-O-{N^{α}-[O-(3-O-methyl-β-L-fucopyranosyl)-4-hydroxy-phenylamino-thiocarbonyl]-N^{ε}-[fluorenyl-9-methoxycarbonyl]-lysyl-leucyl}-camptothecin:

Die Verbindung aus Beispiel 5.1.c wird in Analogie zu Beispiel 1.1 mit 1,2 Äquivalent des Kohlenhydratsderivats aus Beispiel I.1 umgesetzt. Das Rohprodukt wird durch Fallung aus Methanol mit Ether gereinigt. Ausb.: 83 % [DC Acetonitril/Wasser/Eisessig 5:1:0,2 R_{f} = 0,21].

### 5.1) 20(S)-7-[4-Methyl piperazino)methyl]-10,11-(ethylendioxy)-20-O-{N^{α}-[O-(3-O-methyl-β-L-fucopyranosyl)-4-hydroxy-phenylamino-thiocarbonyl]-lysyl-leucyl}-camptothecin, Hydrochlorid

Das Konjugat 5 1 d wird mit Piperidin in DMF deblockiert. Nach 20 min engt man ein und destilliert zweimal mit Dichlormethan nach. Das Produkt wird aus Dichlormethan/Methanol mit Ether gefällt. Anschließend nimmt man in Wasser auf, versetzt mit 1 Aquivalent einer 0,1N HCl-Lösung und lyophilisiert Ausb. 95 % [DC. Acetonitril/Wasser/Eisessig 10·3:1,5 R_{f} = 0,15] [FAB-MS: m/z = 1071 = M+H]

### Beispiel 6.1

### 7-Ethyl-10-hydroxy-20-O-{N^{α}-[O-(3-O-methyl-β-L-fucopyranosyl)-4-hydroxyphenylamino-thiocarbonyl]-lysyl-valyl}-camptothecin, Hydrochlorid

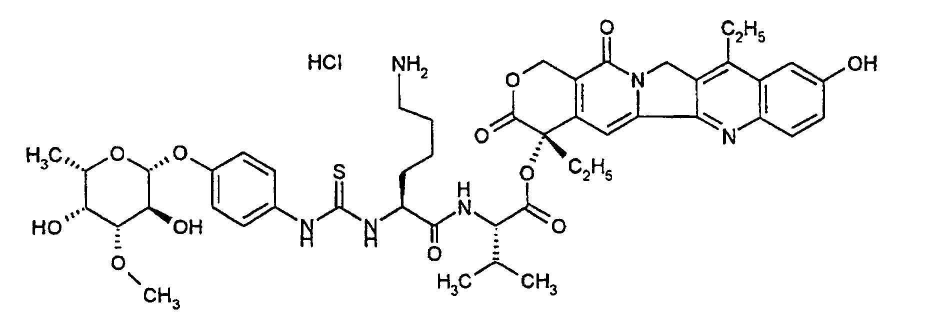

### 6.I.a) 20-O-[N-(tert-Butoxycarbonyl)-valyl]-7-ethyl-10-hydroxy-camptothecin:

Die Verbindung wird unter Verwendung des in 1.1.a beschriebenen Verfahrens aus 392,4 mg (1,0 mmol) 20(S)-7-Ethyl-10-hydroxy-camptothecin (S. Sawada et al., Chem. Pharm. Bull. 39 (1991) 3183-3188) und insgesamt 2,43 g (10,0 mmol) N-(tert-Butoxycarbonyl)-valin-N-Carbonsäurehydrid innerhalb von 6 Tagen hergestellt Man erhält nach Flash-Chromatographie [Petrolether/Ethylacetat 5:1 -> 2:1 -> 1:1] 353 mg (45 %) an hellgelben Kristallen [DC Acetonitril/Ethylacetat 1:1): R_{f} = 0,63; Schmp. = 95-97°C].

### 6.1.b) 7-Ethyl-10-hydroxy-20-0-valyl-camptothecin, Trifluoracetat:

Aus Verbindung 6.1.a (340 mg, 0,43 mmol) wird wie unter 1.1.b beschrieben die N-(tert-Butoxycarbonyl)-Gruppe abgespalten. Man erhält 255 mg (98 %) an gelben Kristallen [DC (Acetonitril/Ethylacetat 1.1): R_{f} = 0,04; Schmp. = 189°C (Zers.)].

### 6.1.c 20-O-[N^{α}-(tert-Butoxycarbonyl)-N^{ε}-(fluorenyl-9-methoxycarbonyl)-lysyl-valyl]-7-ethyl-10-hydroxy-camptothecin:

In Analogie zu 1.1.c werden 562,3 mg (1,2 mmol) N^{α}-(tert-Butoxycarbonyl)-N^{ε}-(fluorenyl-9-methoxycarbonyl)-lysin mit Verbindung 6.1.b (242,2 mg, 0,4 mmol) umgesetzt. Nach Einengen im Vakuum und Reinigung durch Flash-chromatographie [Petrolether/Ethylacetat 5:1 -> 3:1 -> 1.1] erhält man gelbe Kristalle. Ausb.: 251 mg (67 %) [DC (Acetonitril/Ethylacetat 1.1): R_{f} = 0,68; Schmp. = 163°C (Zers.)].

### 6.1.d) 7-Ethyl-20-O-[N^{ε}-(fluorenyl-9-methoxycarbonyl)-lysyl-valyl]-10-hydroxy-camptothecin, Trifluoracetat:

Obige Verbindung (244,9 mg, 0,26 mmol) wird wie beschrieben mit Trifluoressigsäure in Dichlormethan entschützt. Man erhält 115 mg (46 %) an gelben Kristallen [DC (Acetonitril/Ethylacetat 1:1): R_{f} = 0,05; Schmp. = 196°C (Zers.)].

### 6.1.c) 20(S)-7-Ethyl-10-hydroxy-20-O-{N^{α}-[O-(3-O-methyl-β-L-fucopyranosyl)-4-hydroxy-phenylamino-thiocarbonyl]-N^{ε}-[fluorenyl-9-methoxycarbonyl]-lysyl-valyl}-camptothecin:

Die Verbindung aus Beispiel 6.1.d wird in Analogie zu Beispiel 1.1 mit Äquivalenten des Kohlenhydratsderivats aus Beispiel 1:1 umgesetzt und das Produkt durch Flashchromatographie [Petrolether/Ethylacetat 1.1 -> 2:3 -> 1:2] gereinigt. Man erhält in 36 5-iger Ausbeute beige Kristalle [DC (Acetonitril/Ethylacetat 1:1): R_{f} = 0,55]

### 6.1) 20(S)-7-Ethyl-10-hydroxy-20-O-{N^{α}-[O-(3-O-methyl-β-L-fucopyranosyl)-4-hydroxy-phenylamino-thiocarbonyl]-lysyl-valyl}-camptothecin, Hydrochlorid.

Das Konjugat 6.1.e wird mit Piperidin in DMF deblockiert. Nach 2 h engt man ein und fällt den Rückstand zweimal aus Dichlormethan/Methanol 1:1 mit Diethylether um. Anschließend nimmt man in Wasser auf, versetzt mit 1 Äquivalent einer 0,01N HCl-Lösung und lyophilisiert.

## Patentansprüche

1. Verbindungen der allgemeinen Formel (I)
A-Cp-B (I)
worin
Cp für eine Gruppe der Formeln 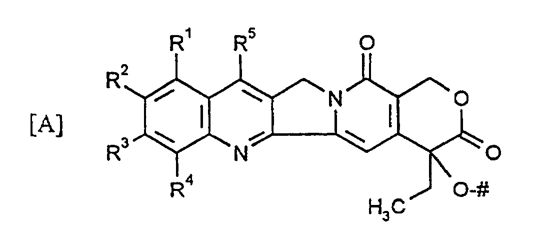 worin
R¹, R², R³ und R⁴ unabhängig voneinander Wasserstoff, Alkyl mit bis zu 3 Kohlenstoffatomen, Halogen, Amino, Hydroxy oder Nitro bedeuten können oder
R² und R³ zusammen eine Gruppe der Formel 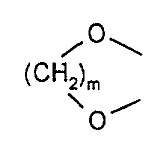 bedeuten, wobei
m die Werte 1 oder 2 annehmen kann, und
R⁵ für H, -CH₂CH₃, -CH₂-O-*, -CH₂-NH*, 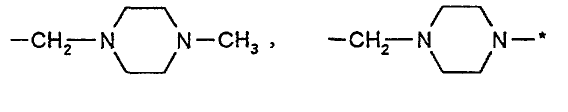 steht,
oder für -CH₂-N(CH₂CH₃)R⁶ oder 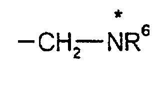 steht,
worin R⁶ Arylmethyl oder Hetarylmethyl bedeutet, 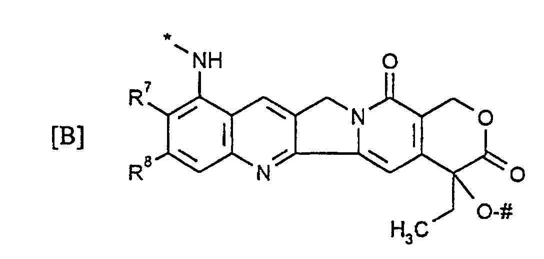 worin
R⁷ und R⁸ wie R² und R³ definiert sind und mit diesen gleich oder verschieden sein können, 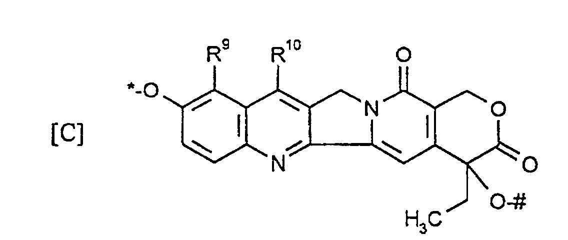 worin
R⁹ für Wasserstoff oder -CH₂-N(CH₃)₂ steht und
R¹⁰ für Wasserstoff oder Ethyl steht,
oder 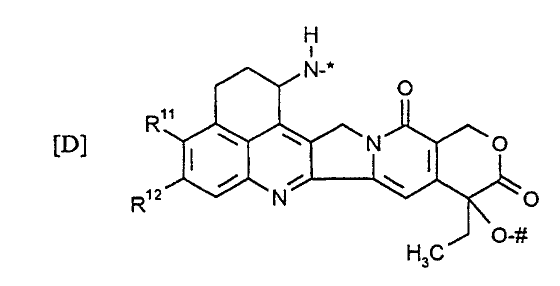 worin
R¹¹ und R¹² wie R² und R³ definiert sind und mit diesen gleich oder verschieden sein können,
oder 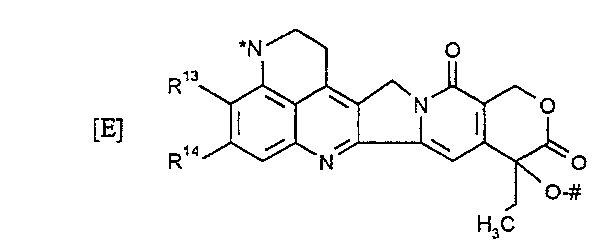 worin
R¹³ und R¹⁴ wie R² und R³ definiert sind und mit diesen gleich oder verschieden sein können,
steht, wobei Cp an den mit # markierten Positionen mit A und an den mit * markierten Positionen mit B verknüpft ist,
A einen Rest der Formel 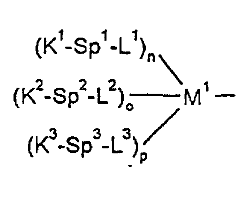 bedeutet, wobei gilt 1 ≤ (n + o + p) ≤ 3,
B Wasserstoff oder einen Rest der Formel 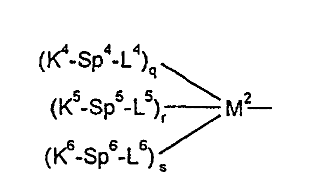 bedeutet, wobei gilt 0 ≤ (q + r + s) ≤ 3, worin
M¹ und M² unabhängig voneinander für eine Brücken-Gruppierung stehen, deren Hauptkette bis zu 21 Atome in linearer Abfolge umfaßt,
L¹, L², L³, L⁴, L⁵ und L⁶ unabhängig voneinander für Linker-Gruppierungen stehen, wie sie in der Glycokonjugatchemie gängigerweise eingesetzt werden,
Sp¹, Sp², Sp³, Sp⁴, Sp⁵ und Sp⁶ unabhängig voneinander für Arylen mit bis zu 10 Kohlenstoffatomen oder für Alkylen mit bis zu 8 Kohlenstoffatomen stehen, die jeweils gegebenenfalls substituiert sind, und
K¹, K², K³, K⁴, K⁵ und K⁶ unabhängig voneinander für einen Rest der Formel (II) 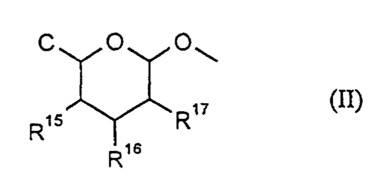 stehen, worin
C Methyl, Hydroxymethyl, Alkoxymethyl mit bis zu 6 Kohlenstoffatomen, Acyloxymethyl mit bis zu 6 Kohlenstoffatomen oder einen Rest der Formel -CH₂-D bedeutet, worin
D für einen Rest der Formel (II) steht,
R¹⁵, R¹⁶ und R¹⁷ unabhängig voneinander Wasserstoff, Hydroxy, gegebenenfalls durch Hydroxyl substituiertes Alkoxy mit bis zu 6 Kohlenstoffatomen, gegebenenfalls durch Alkyl oder Acyl mit bis zu 6 Kohlenstoffatomen substituiertes Amino, Halogen, Sulfat oder eine Gruppe der Formeln 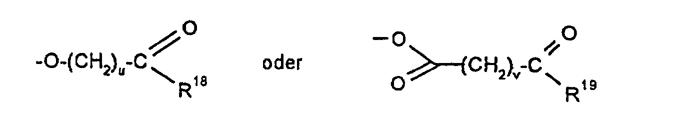
bedeuten, worin
R¹⁸ und R¹⁹ unabhängig voneinander für Hydroxyl oder Alkoxy mit bis zu 6 Kohlenstoffatomen stehen oder für Amino, das gegebenenfalls durch Alkyl mit bis zu 6 Kohlenstoffatomen substituiert ist, stehen und
u und v unabhängig voneinander die Werte 0, 1, 2, 3 oder 4 annehmen können
oder
R¹⁵, R¹⁶ und R¹⁷ unabhängig voneinander für einen Rest der Formel (II) stehen
oder
zwei der Reste R¹⁵, R¹⁶, R¹⁷ zusammen für eine Epoxygruppe stehen,
sowie deren Isomere, Isomerengemische und Salze mit der Maßgabe, dass R¹, R², R³, R⁴ und R⁵ nicht gleichzeitig Wasserstoff sein können.

2. Verbindungen der allgemeinen Formel (I) gemäß Anspruch 1, worin K¹, K², K³, K⁴, K⁵ und K⁶ unabhängig voneinander für einen Rest der Formel (II) stehen können, wobei
C Methyl, Hydroxymethyl, Methoxymethyl oder Acetoxymethyl bedeutet,
R¹⁵ Wasserstoff, Hydroxyl, Methoxy oder eine Gruppe der Formeln 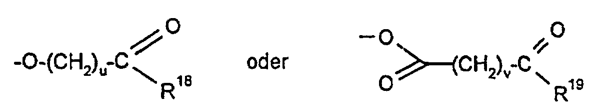
bedeutet, worin
u und v unabhängig voneinander die Werte 1 oder 2 annehmen können und
R¹⁸ und R¹⁹ unabhängig voneinander für Hydroxyl oder Alkoxy mit bis zu 4 Kohlenstoffatomen stehen,
oder
R¹⁵ für einen Rest der Formel (II) steht,
R¹⁶ Wasserstoff, Hydroxyl, Halogen, Alkoxy mit bis zu 4 Kohlenstoffatomen, Sulfat oder eine Gruppe der Formeln 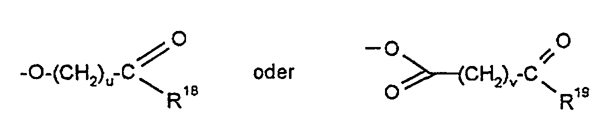 bedeutet, worin
u und v unabhängig voneinander die Werte 1 oder 2 annehmen können und
R¹⁸ und R¹⁹ unabhängig voneinander für Hydroxy oder Alkoxy mit bis zu 4 Kohlenstoffatomen stehen oder für Amino, das gegebenenfalls durch Alkyl mit bis zu 4 Kohlenstoffatomen substituiert ist, stehen,
R¹⁷ Hydroxyl, Alkoxy mit bis zu 4 Kohlenstoffatomen, das gegebenenfalls durch Hydroxy substituiert ist, gegebenenfalls durch Alkyl oder Acyl mit bis zu 4 Kohlenstoffatomen substituiertes Amino, oder eine Gruppe der Formeln 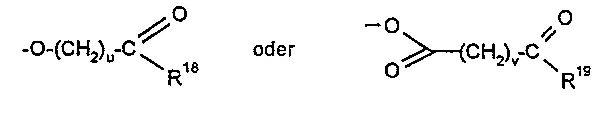 bedeutet, worin
u und v unabhängig voneinander die Werte 1 oder 2 annehmen können und
R¹⁸ und R¹⁹ unabhängig voneinander für Hydroxyl oder Alkoxy mit bis zu 4 Kohlenstoffatomen stehen,
oder worin
R¹⁵ und R¹⁶ gemeinsam für eine Epoxygruppe stehen,
sowie deren Isomere, Isomerengemische und Salze.

3. Verbindungen der allgemeinen Formel (I) gemäß Anspruch 1, worin Sp¹, Sp², Sp³, Sp⁴. Sp⁵ und Sp⁶ unabhängig voneinander für Arylen mit bis zu 10 Kohlenstoffatomen stehen können, das mit je einer Gruppe K¹, K², K³, K⁴, K⁵ oder K⁶ und L¹, L², L³, L⁴, L⁵ oder L⁶ verknüpft ist und das gegebenenfalls noch ein- oder mehrfach durch Hydroxy, Carboxy, Carboxyalkyl mit bis zu 4 Kohlenstoffatomen, Nitro, Cyano, Halogen, Alkyl mit bis zu 4 Kohlenstoffatomen, Halogenalkyl mit bis zu 4 Kohlenstoffatomen oder durch Alkoxy mit bis zu 4 Kohlenstoffatomen substituiert ist, sowie deren Isomere, Isomerengemische und Salze.

4. Verbindungen der allgemeinen Formel (I) gemäß Anspruch 1, worin
L¹, L², L³, L⁴, L⁵ und L⁶ unabhängig voneinander 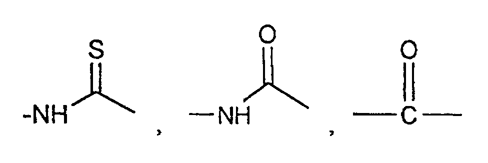 oder 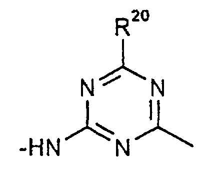 bedeuten, wobei
R²⁰ für Chlor oder für Hydroxyalkylamino mit bis zu 6 Kohlenstoffatomen steht,
sowie deren Isomere, Isomerengemische und Salze.

5. Verbindungen der allgemeinen Formel (I) gemäß Anspruch 1, worin M¹ und M² unabhängig voneinander für ein Peptid stehen können, das über eine Aminofunktion mit L¹, L², L³, L⁴, L⁵ und/oder L⁶ verknüpft ist, über eine Acylfunktion mit Cp verknüpft ist und dessen Aminosäurebausteine gegebenenfalls Schutzgruppen tragen können, sowie deren Isomere, Isomerengemische und Salze.

6. Verbindungen gemäß Anspruch 5, **dadurch gekennzeichnet, daß** M₁ und/oder M₂ für ein Mono-, Di- oder Tripeptid stehen.

7. Verbindungen gemäß Anspruch 5 oder 6, **dadurch gekennzeichnet, daß** das Peptid aus gegebenenfalls Schutzgruppen tragenden Aminosäurebausteinen besteht, ausgewählt aus der Gruppe bestehend aus Glycyl, Alanyl, Valyl, Leucyl, Lysyl, Seryl, Glutamyl, Threonyl, Asparagyl, Isoleucyl, Diaminopropionyl, Diaminobutyryl, Arginyl, Histidyl und/oder Ornithyl.

8. Verfahren zur Herstellung von Verbindungen der allgemeinen Formel (I) gemäß Anspruch 1, **dadurch gekennzeichnet, daß** man Verbindungen der allgemeinen Formel (III)
H_{B}-Cp-H_{A} (III)
worin Cp die oben angegebene Bedeutung hat und das Wasserstoffatom H_{A} an der mit # und H_{B} an der mit * bezeichneten Position sitzt, gegebenenfalls nach Ersatz von H_{B} durch eine Schutzgruppe, mit einer dem oben definierten Rest M¹ entsprechenden aktivierten Carboxylkomponente M¹a, die gegebenenfalls Schutzgruppen trägt, in einem geeigneten Lösungsmittel, gegebenenfalls in Gegenwart einer Base, nach üblichen Methoden umsetzt, gegebenenfalls mittels bekannter Methoden selektiv eine, mehrere oder alle Schutzgruppen von M¹ abspaltet und das Produkt mit Verbindungen der allgemeinen Formel (IV)
K¹-Sp¹-L¹a (IV)
worin K¹ und Sp¹ wie oben definiert sind und L¹a für eine reaktive Vorstufe der Gruppe L¹ steht, umsetzt, wobei gegebenenfalls die Schutzgruppen selektiv abgespalten und schrittweise verschiedene Gruppen K²-Sp²-L²- und K³-Sp³-L³- in vergleichbarer Weise eingeführt werden können, und daß man, falls mit der mit * bezeichneten Position eine Kohlenhydrat-Komponente verknüpft werden soll, gegebenenfalls selektiv die Schutzgruppe, die H_{B} ersetzt, mittels bekannter Methoden abspaltet, und in der oben beschriebenen Weise den Rest M² und wie gewünscht Reste der Formeln K⁴-Sp⁴-L⁴-, K⁵-Sp⁵-L⁵- und K⁶-Sp⁶-L⁶- einführt
oder daß man, falls M¹ und/oder M² ein Peptid bedeuten, in vergleichbarer Weise nach üblichen Methoden einen ersten Aminosäurerest in Form der entsprechenden gegebenenfalls Schutzgruppen tragenden Carboxylkomponente einführt, gegebenenfalls Schutzgruppen abspaltet, weiterhin gegebenenfalls Schutzgruppen tragende Aminosäurereste anknüpft, gegebenenfalls erneut Schutzgruppen abspaltet, wie oben angegeben Reste der Formeln K¹-Sp¹-L¹-, K²-Sp²-L²-, K³-Sp³-L³-, K⁴-Sp⁴-L⁴-, K⁵-Sp⁵-L⁵-und/oder K⁶-Sp⁶-L⁶- einführt und falls erforderlich Schutzgruppen abspaltet,
und daß man weiterhin gegebenenfalls nach üblichen Methoden die Stereoisomeren trennt und daß man gegebenenfalls die Verbindungen in ihre Salze überführt.

9. Verwendung der Verbindungen der allgemeinen Formel (I) gemäß Anspruch 1 zur Herstellung von Arzneimitteln.

10. Arzneimittel, enthaltend Verbindungen der allgemeinen Formel (I) gemäß Anspruch 1.

## Claims

1. Compounds of the general formula (I)
A-Cp-B (I)
in which
Cp represents a group of the formulae 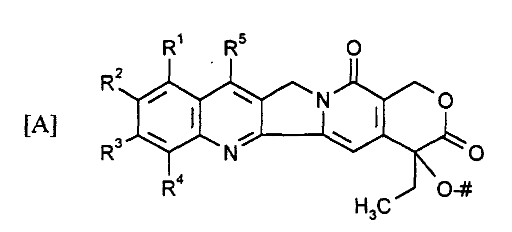 in which
R¹, R², R³ and R⁴ independently of one another may represent hydrogen, alkyl having up to 3 carbon atoms, halogen, amino, hydroxyl or nitro or
R² and R³ together represent a group of the formula 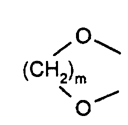 where
m may have the values 1 or 2 and
R⁵ represents H, -CH₂CH₃, -CH₂-O-*, -CH₂-NH*, 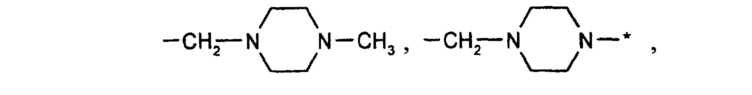 or represents -CH₂-N(CH₂CH₃)R⁶ or 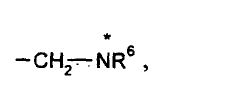 in which R⁶ represents arylmethyl or hetarylmethyl, 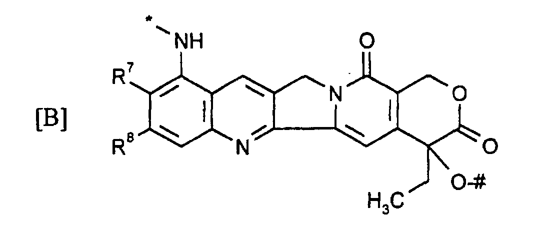 in which
R⁷ and R⁸ are as defined for R² and R³ and may be identical or different to these, 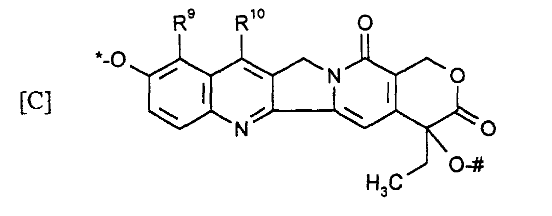 in which
R⁹ represents hydrogen or -CH₂-N(CH₃)₂ and
R¹⁰ represents hydrogen or ethyl,
or 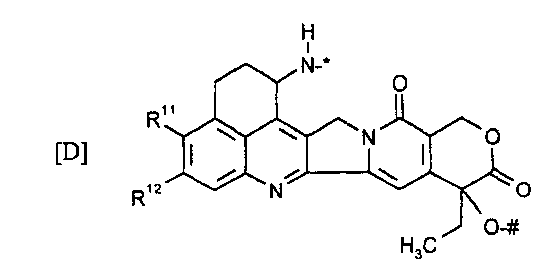 in which
R¹¹ and R¹² ² are as defined for R² and R³ and may be identical or different to these,
or 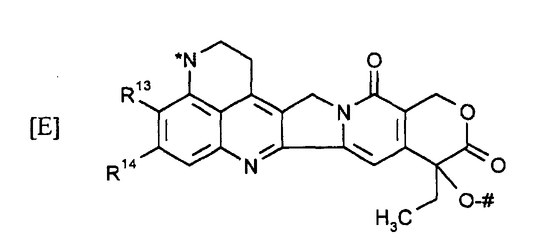 in which
R¹³ and R¹⁴ are as defined for R² and R³ and may be identical or different to these,
where Cp is attached to A on the positions labelled # and attached to B on the positions labelled *,
A represents a radical of the formula 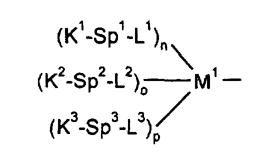 where 1 ≤ (n + o + p) ≤ 3,
B represents hydrogen or a radical of the formula 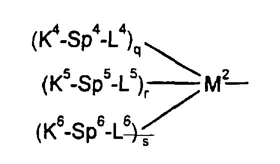 where 0 ≤ (q + r + s) ≤ 3, in which
M¹ and M² independently of one another each represent a bridge grouping whose main chain includes up to 21 atoms in linear order,
L¹, L², L³, L⁴, L⁵ and L⁶ independently of one another each represent linker groupings customarily used in glycoconjugate chemistry,
Sp¹, Sp², Sp³, Sp⁴, Sp⁵ and Sp⁶ independently of one another each represent arylene having up to 10 carbon atoms or represent alkylene having up to 8 carbon atoms which are in each case optionally substituted, and
K¹, K², K³, K⁴, K⁵ and K⁶ independently of one another each represent a radical of the formula (II) 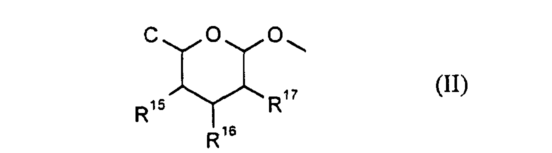 in which
C represents methyl, hydroxymethyl, alkoxymethyl having up to 6 carbon atoms, acyloxymethyl having up to 6 carbon atoms or a radical of the formula -CH₂-D in which
D represents a radical of the formula (II),
R¹⁵, R¹⁶ and R¹⁷ independently of one another each represent hydrogen, hydroxyl, optionally hydroxyl-substituted alkoxy having up to 6 carbon atoms, amino which is optionally substituted by alkyl or acyl having up to 6 carbon atoms, halogen, sulphate or a group of the formula 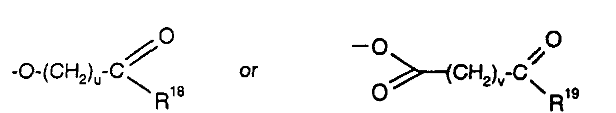 in which
R¹⁸ and R¹⁹ independently of one another each represent hydroxyl or alkoxy having up to 6 carbon atoms or represent amino which is optionally substituted by alkyl having up to 6 carbon atoms, and
u and v independently of one another may each have the values 0, 1, 2, 3 or 4,
or
R¹⁵, R¹⁶ and R¹⁷ independently of one another each represent a radical of the formula (II)
or
two of the radicals R¹⁵, R¹⁶, R¹⁷ together represent an epoxy group,
and their isomers, isomer mixtures and salts, with the proviso that R¹, R², R³, R⁴ and R⁵ cannot simultaneously be hydrogen.

2. Compounds of the general formula (I) according to Claim 1, in which K¹, K², K³, K⁴, K⁵ and K⁶ independently of one another may each represent a radical of the formula (II) where
C represents methyl, hydroxymethyl, methoxymethyl or acetoxymethyl,
R¹⁵ represents hydrogen, hydroxyl, methoxy or a group of the formula 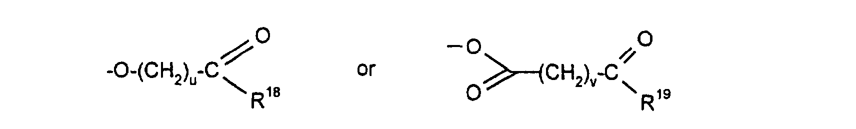 in which
u and v independently of one another may each have the values 1 or 2 and
R¹⁸ and R¹⁹ independently of one another each represent hydroxyl or alkoxy having up to 4 carbon atoms,
or
R¹⁵ represents a radical of the formula (II),
R¹⁶ represents hydrogen, hydroxyl, halogen, alkoxy having up to 4 carbon atoms, sulphate or a group of the formula 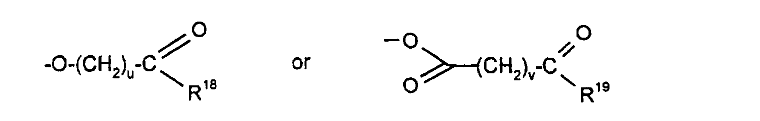 in which
u and v independently of one another may each have the values 1 or 2 and
R¹⁸ and R¹⁹ independently of one another each represent hydroxyl or alkoxy having up to 4 carbon atoms or represent amino which is optionally substituted by alkyl having up to 4 carbon atoms,
R¹⁷ represents hydroxyl, alkoxy having up to 4 carbon atoms which is optionally substituted by hydroxyl, amino which is optionally substituted by alkyl or acyl having up to 4 carbon atoms, or a group of the formula 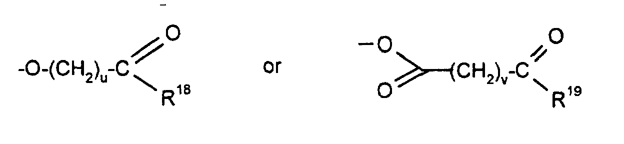 in which
u and v independently of one another may each have the values 1 or 2 and
R¹⁸ and R¹⁹ independently of one another each represent hydroxyl or alkoxy having up to 4 carbon atoms,
or in which
R¹⁵ and R¹⁶ together represent an epoxy group,
and their isomers, isomer mixtures and salts.

3. Compounds of the general formula (I) according to Claim 1 in which Sp¹, Sp², Sp³, Sp⁴, Sp⁵ and Sp⁶ independently of one another may each represent arylene having up to 10 carbon atoms which is attached to in each case one group K¹, K², K³, K⁴, K⁵ or K⁶ and L¹, L², L³, L⁴, L⁵ or L⁶ and which is optionally also mono- or polysubstituted by hydroxyl, carboxyl, carboxyalkyl having up to 4 carbon atoms, nitro, cyano, halogen, alkyl having up to 4 carbon atoms, halogenoalkyl having up to 4 carbon atoms or by alkoxy having up to 4 carbon atoms, and their isomers, isomer mixtures and salts.

4. Compounds of the general formula (I) according to Claim 1 in which
L¹, L², L³, L⁴, L⁵ and L⁶ independently of one another each represent 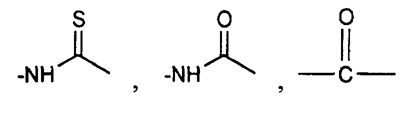 or 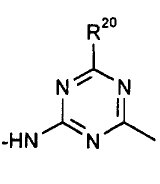 where
R²⁰ represents chlorine or represents hydroxyalkylamino having up to 6 carbon atoms,
and their isomers, isomer mixtures and salts.

5. Compounds of the general formula (I) according to Claim 1 in which M¹ and M² independently of one another may each represent a peptide which is attached to L¹, L², L³, L⁴, L⁵ and/or L⁶ via an amino function, is attached to Cp via an acyl function and whose amino acid building blocks may optionally carry protective groups, and their isomers, isomer mixtures and salts.

6. Compounds according to Claim 5, **characterized in that** M₁ and/or M₂ represent a mono-, di- or tripeptide.

7. Compounds according to Claim 5 or 6, **characterized in that** the peptide comprises amino acid building blocks selected from the group consisting of glycyl, alanyl, valyl, leucyl, lysyl, seryl, glutamyl, threonyl, asparagyl, isoleucyl, diaminopropionyl, diaminobutyryl, arginyl, histidyl and/or ornithyl which optionally carry protective groups.

8. Process for preparing compounds of the general formula (I) according to Claim 1, **characterized in that** compounds of the general formula (III)
H_{B}-Cp-H_{A} (III)
in which Cp is as defined above and the hydrogen atoms H_{A} and H_{B} are located on the positions labelled # and *, respectively, are reacted, if appropriate after replacing H_{B} by a protective group, with an activated carboxyl component M¹ a which corresponds to the radical M' defined above and optionally carries protective groups, in a suitable solvent, if appropriate in the presence of a base, by customary methods, one, more than one or all protective groups of M¹ are, if appropriate, selectively removed by known methods and the product is reacted with compounds of the general formula (IV)
K¹-Sp¹-L¹a (IV)
in which K¹ and Sp' are each as defined above and L¹a represents a reactive precursor of the group L', where the protective groups are, if appropriate, selectively removed and various groups K²-Sp²-L²- and K³-Sp³-L³- can be introduced stepwise in a comparable manner, and that, if a carbohydrate component is to be attached to the position labelled *, the protective group which replaces H_{B} is, if appropriate, selectively removed by known methods and the radical M² and, as desired, radicals of the formulae K⁴-Sp⁴-L⁴-, K⁵-Sp⁵-L⁵- and K⁶-Sp⁶-L⁶- are introduced in the manner described above
or that, if M' and/or M² are a peptide, a first amino acid radical is introduced in a comparable manner by customary methods in the form of a corresponding carboxyl component which optionally carries protective groups, protective groups are, if appropriate, removed, amino acid radicals which optionally carry protective groups are attached, protective groups are, if appropriate, removed again, the abovementioned radicals of the formulae K¹-Sp¹-L¹-, K²-Sp²-L²-, K³-Sp³-L³-, K⁴-Sp⁴-L⁴-, K⁵-Sp⁵-L⁵- and/or K6-Sp6-L6- are introduced and, if required, protective groups are removed,
and that furthermore, if required, the stereoisomers are separated by customary methods and the compounds are, if required, converted into their salts.

9. Use of compounds of the general formula (I) according to Claim 1 for preparing medicaments.

10. Medicaments, comprising compounds of the general formula (I) according to Claim 1.

## Revendications

1. Composés de formule générale (I)
A-Cp-B (I)
dans laquelle
Cp est un groupe de formules 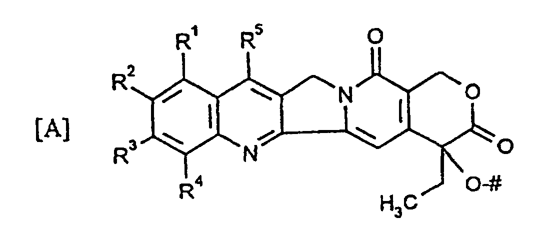 dans laquelle
R¹, R², R³ et R⁴ peuvent représenter, indépendamment les uns des autres, l'hydrogène, un groupe alkyle ayant jusqu'à 3 atomes de carbone, un halogène, un groupe amino, hydroxy ou nitro ou bien
R² et R³ forment ensemble un groupe de formule 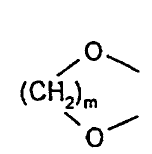 dans laquelle
m peut prendre la valeur 1 ou 2, et
R⁵ représente H, -CH₂CH₃, -CH₂-O-*, -CH₂-NH*, 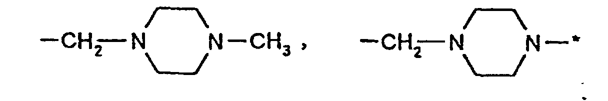 ou bien
-CH₂-N(CH₂CH₃)R⁶
ou 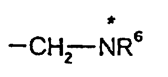 où R⁶ est un groupe arylméthyle ou hétarylméthyle, 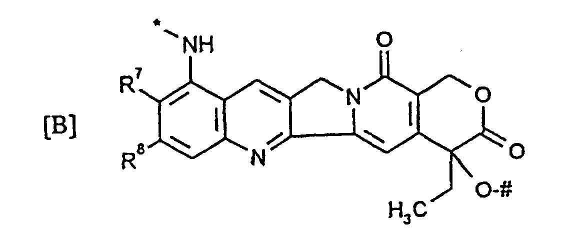 dans laquelle
R⁷ et R⁸ ont la même définition que R² et R³ et peuvent y être identiques ou en être différents, 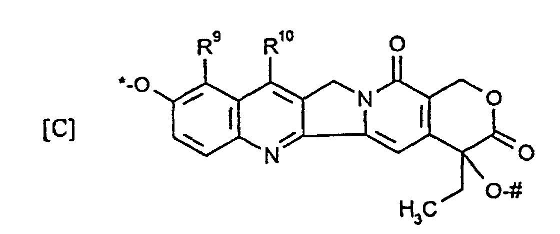 où
R⁹ représente l'hydrogène ou un groupe -CH₂-N(CH₃)₂ et
R¹⁰ représente l'hydrogène ou un groupe éthyle, ou 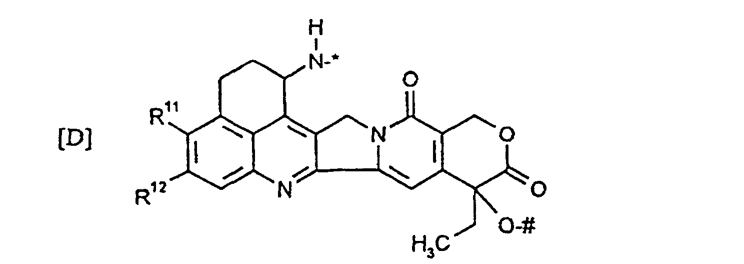 dans laquelle
R¹¹ et R¹² ont la même définition que R² et R³ et peuvent y être identiques ou en être différents, ou 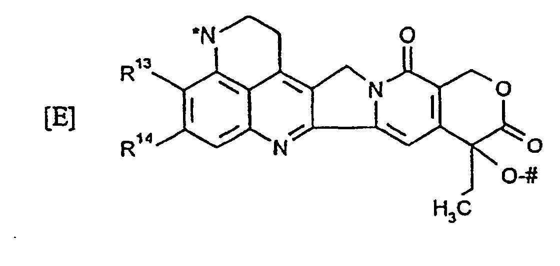 dans laquelle
R¹³ et R¹⁴ ont la même définition que R² et R³ et peuvent y être identiques ou en être différents,
Cp étant lié à A dans les positions marquées d'un signe # et à B dans les positions marquées d'un signe *,
A est un reste de formule 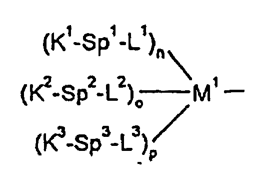 dans laquelle 1 ≤ (n + o + p) ≤ 3,
B représente l'hydrogène ou un reste de formule 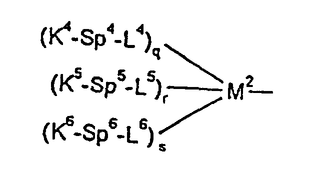 dans laquelle 0 ≤ (q + r + s) ≤ 3,
M¹ et M² représentent, indépendamment l'un de l'autre, un groupement de pontage dont la chaîne principale comprend jusqu'à 21 atomes en succession linéaire,
L¹, L², L³, L⁴, L⁵ et L⁶ représentent, indépendamment les uns des autres, des groupements de jonction semblables à ceux qui sont utilisés ordinairement dans la chimie des glycoconjugués,
Sp¹, Sp², Sp³, Sp⁴, Sp⁵ et Sp⁶ représentent, indépendamment les uns des autres, des groupes arylène ayant jusqu'à 10 atomes de carbone ou des groupes alkylène ayant jusqu'à 8 atomes de carbone, dont chacun est éventuellement substitué, et
K¹, K², K³, K⁴, K⁵ et K⁶ représentent, indépendamment les uns des autres, un reste de formule (II) 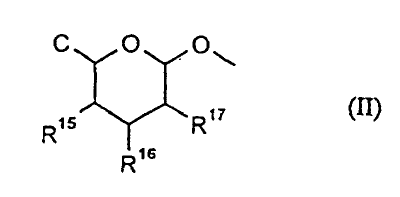 dans laquelle
C est un groupe méthyle, hydroxyméthyle, alkoxyméthyle ayant jusqu'à 6 atomes de carbone, acyloxyméthyle ayant jusqu'à 6 atomes de carbone ou un reste de formule-CH₂-D, dans laquelle
D représente un reste de formule (II),
R¹⁵, R¹⁶ et R¹⁷ représentent, indépendamment les uns des autres, l'hydrogène, un groupe hydroxy, un groupe alkoxy ayant jusqu'à 6 atomes de carbone éventuellement substitué par un radical hydroxyle, un groupe amino éventuellement substitué par un radical alkyle ou acyle ayant jusqu'à 6 atomes de carbone, un halogène, un ion sulfate ou un groupe de formules 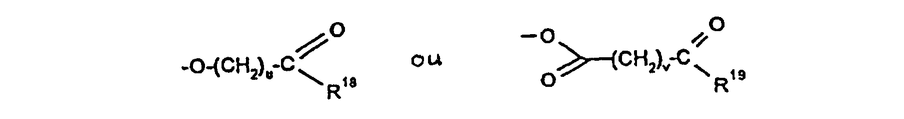 dans lesquelles
R¹⁸ et R¹⁹ représentent, indépendamment l'un de l'autre, un groupe hydroxyle ou un groupe alkoxy ayant jusqu'à 6 atomes de carbone, ou un groupe amino qui est éventuellement substitué par un radical alkyle ayant jusqu'à 6 atomes de carbone, et
u et v peuvent prendre, indépendamment l'un de l'autre, les valeurs 0, 1, 2, 3 ou 4
ou bien
R¹⁵, R¹⁶ et R¹⁷ représentent, indépendamment les uns des autres, un reste de formule (II)
ou bien
deux des restes R¹⁵, R¹⁶, R¹⁷ forment ensemble un groupe époxy,
ainsi que leurs isomères, leurs mélanges d'isomères et leurs sels,
sous réserve que R¹, R², R³, R⁴ et R⁵ ne puissent pas représenter simultanément de l'hydrogène.

2. Composés de formule générale (I) suivant la revendication 1, dans laquelle K¹, K², K³, K⁴, K⁵ et K⁶ peuvent représenter, indépendamment les uns des autres, un reste de formule (II), dans laquelle
C est un groupe méthyle, hydroxyméthyle, méthoxyméthyle ou acétoxyméthyle,
R¹⁵ représente l'hydrogène, un groupe hydroxyle, méthoxy ou un groupe de formules 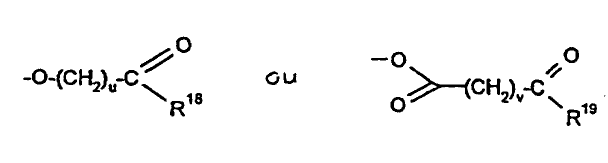 dans lesquelles
u et v peuvent prendre, indépendamment l'un de l'autre, les valeurs 1 ou 2 et
R¹⁸ et R¹⁹ représentent, indépendamment l'un de l'autre, un groupe hydroxyle ou un groupe alkoxy ayant jusqu'à 4 atomes de carbone,
ou bien
R¹⁵ représente un reste de formule (II),
R¹⁶ représente l'hydrogène, un groupe hydroxyle, un halogène, un groupe alkoxy ayant jusqu'à 4 atomes de carbone, un ion sulfate ou un groupe de formules 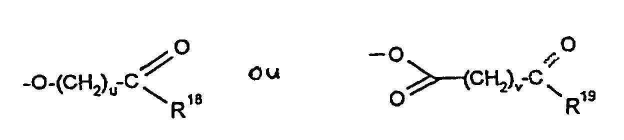 dans lesquelles
u et v peuvent prendre, indépendamment l'un de l'autre, les valeurs 1 ou 2 et
R¹⁸ et R¹⁹ représentent, indépendamment l'un de l'autre, un groupe hydroxy ou alkoxy ayant jusqu'à 4 atomes de carbone ou un groupe amino qui est éventuellement substitué par un radical alkyle ayant jusqu'à 4 atomes de carbone,
R¹⁷ est un groupe hydroxyle, alkoxy ayant jusqu'à 4 atomes de carbone, qui est éventuellement substitué par un radical hydroxy, amino éventuellement substitué par un radical alkyle ou acyle ayant jusqu'à 4 atomes de carbone, ou un groupe de formules 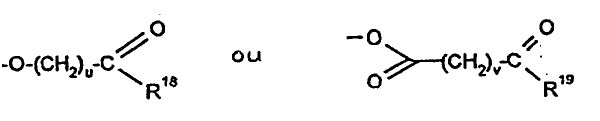 dans lesquelles
u et v peuvent prendre, indépendamment l'un de l'autre, les valeurs 1 ou 2 et
R¹⁸ et R¹⁹ représentent, indépendamment l'un de l'autre, un groupe hydroxyle ou alkoxy ayant jusqu'à 4 atomes de carbone,
ou dans laquelle
R¹⁵ et R¹⁶ forment ensemble un groupe époxy,
ainsi que leurs isomères, leurs mélanges d'isomères et leurs sels.

3. Composés de formule générale (I) suivant la revendication 1, dans laquelle Sp¹, Sp², Sp³, Sp⁴, Sp⁵ et Sp⁶ peuvent représenter, indépendamment les uns des autres, un groupe arylène ayant jusqu'à 10 atomes de carbone, qui est lié avec chaque fois un groupe K¹, K², K³, K⁴, K⁵ ou K⁶ et chaque fois un groupe L¹, L², L³, L⁴, L⁵ ou L⁶ et qui est encore substitué une ou plusieurs fois par un radical hydroxy, carboxy, carboxyalkyle ayant jusqu'à 4 atomes de carbone, nitro, cyano, halogéno, alkyle ayant jusqu'à 4 atomes de carbone, halogénalkyle ayant jusqu'à 4 atomes de carbone ou par un radical alkoxy ayant jusqu'à 4 atomes de carbone, ainsi que leurs isomères, leurs mélanges d'isomères et leurs sels.

4. Composés de formule générale (I) suivant la revendication 1, où
L¹, L², L³, L⁴, L⁵ et L⁶ représentent, indépendamment les uns des autres, un groupe 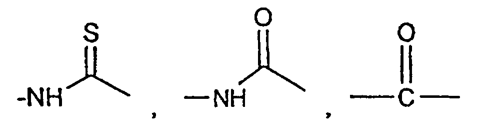 ou 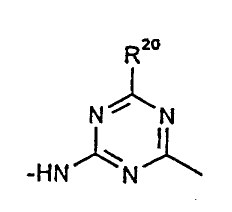 où
R²⁰ représente du chlore ou un groupe hydroxyalkylamino ayant jusqu'à 6 atomes de carbone,
ainsi que leurs isomères, leurs mélanges d'isomères et leurs sels.

5. Composés de formule générale (I) suivant la revendication 1, où M¹ et M² peuvent représenter, indépendamment l'un de l'autre, un peptide qui est lié par l'intermédiaire d'une fonction amino à L¹, L², L³, L⁴, L⁵ et/ou L⁶, qui est lié par l'intermédiaire d'une fonction acyle à Cp et dont les motifs amino-acides structuraux peuvent porter éventuellement des groupes protecteurs, ainsi que leurs isomères, leurs mélanges d'isomères et leurs sels.

6. Composés suivant la revendication 5, **caractérisés en ce que** M¹ et/ou M² représentent un monopeptide, un dipeptide ou un tripeptide.

7. Composés suivant la revendication 5 ou 6, **caractérisés en ce que** le peptide est constitué de motifs amino-acides structuraux éventuellement porteurs de groupes protecteurs, choisis dans le groupe consistant en motifs glycyle, alanyle, valyle, leucyle, lysyle, séryle, glutamyle, thréonyle, asparagyle, isoleucyle, diaminopropionyle, diaminobutyryle, arginyle, histidyle et/ou ornithyle.

8. Procédé de production de composés de formule générale (I) suivant la revendication 1, **caractérisé en ce qu'**on fait réagir selon des modes opératoires classiques des composés de formule générale (III)
H_{B}-Cp-H_{A} (III)
dans laquelle Cp a la définition indiquée ci-dessus et l'atome d'hydrogène H_{A} se trouve dans la position marquée d'un signe # et l'atome d'hydrogène H_{B} se trouve dans la position marquée d'un signe *, éventuellement après remplacement de H_{B} par un groupe protecteur, avec un composant carboxylé M¹a activé correspondant au reste M¹ défini ci-dessus, qui porte éventuellement des groupes protecteurs, dans un solvant approprié, éventuellement en présence d'une base, le cas échéant on élimine sélectivement par des modes opératoires connus l'un, plusieurs ou la totalité des groupes protecteurs de M¹ et on fait réagir le produit avec des composés de formule générale (IV)
K¹-Sp¹-L¹a (IV)
dans laquelle K¹ et Sp¹ sont tels que définis ci-dessus et L¹a représente un précurseur réactif du groupe L¹, avec éventuellement possibilité d'élimination sélective des groupes protecteurs et introduction progressive de divers groupes K²-Sp²-L²- et K³-Sp³-L³- de manière comparable, et **en ce que**, si un composant glucidique doit être lié à la position marquée d'un signe *, on élimine, le cas échéant, sélectivement selon des modes opératoires connus le groupe protecteur qui remplace H_{B} et on introduit de la manière décrite ci-dessus le reste M² et, de la manière souhaitée, des restes de formules K⁴-Sp⁴-L⁴-, K⁵-Sp⁵-L⁵- et K⁶-Sp⁶-L⁶-,
ou **en ce que** si M¹ et/ou M² désignent un peptide, on introduit de manière comparable selon des modes opératoires connus un premier reste d'amino-acide sous forme du composant carboxylé correspondant portant éventuellement des groupes protecteurs, on élimine éventuellement les groupes protecteurs, on lie en outre des restes d'amino-acides portant éventuellement des groupes protecteurs, on élimine de nouveau éventuellement les groupes protecteurs, on introduit comme indiqué ci-dessus des restes de formules K¹-Sp¹-L¹-, K²-Sp²-L²-, K³-Sp³-L³-, K⁴-Sp⁴-L⁴-, K⁵-Sp⁵-L⁵- et/ou K⁶-Sp⁶-L⁶- et, si nécessaire, on élimine les groupes protecteurs,
et **en ce qu'**on sépare en outre éventuellement par des modes opératoires classiques les stéréo-isomères, et **en ce qu'**on transforme éventuellement les composés en leurs sels.

9. Utilisation des composés de formule générale (I) suivant la revendication 1 pour la préparation de médicaments.

10. Médicaments contenant des composés de formule générale (I) suivant la revendication 1.
